# EUROPEAN PATENT SPECIFICATION

(11) **EP 1 664 309 B1**
(45) Date of publication and mention of the grant of the patent: **11.11.2009**
(21) Application number: 04766699.5
(22) Date of filing: 03.09.2004
(51) Int. Cl.: C12N 15/82, C12Q 1/48, C12N 9/12, A01H 5/00

(54) **PLANTS HAVING IMPROVED GROWTH CHARACTERISTICS AND METHOD FOR MAKING THE SAME**
PFLANZEN MIT VERBESSERTEN WACHSTUMSEIGENSCHAFTEN SOWIE VERFAHREN ZU DEREN HERSTELLUNG
PLANTES PRESENTANT DES CHARACTERISTIQUES DE CROISSANCE AMELIORÉES ET PROCÉDÉ DE FABRICATION

(30) Priority: 05.09.2003 EP 03077811
(43) Date of publication of application: 07.06.2006
(73) Proprietor: CropDesign N.V., 9052 Zwijnaarde (BE)
(72) Inventor: BROEKAERT, Willem, B-1700 Dilbeek (BE); FRANKARD, Valerie, B-1640 Sint-Genesius-Rode (BE); HATZFELD, Yves, F-59000 Lille (FR); MIRONOV, Vladimir, B-9000 Gent (BE)
(74) Representative: Mistry, Meeta
(86) International application number: PCT/EP2004/052035
(87) International publication number: WO 2005/024029

(56) References cited:
- WO-A-00/56905
- WO-A-02/16655
- WO-A-98/41642
- WO-A-2004/035798
- PORCEDDU ANDREA ET AL: "A plant-specific cyclin-dependent kinase is involved in the control of G2/M progression in plants" JOURNAL OF BIOLOGICAL CHEMISTRY, vol. 276, no. 39, 28 September 2001 (2001-09-28), pages 36354-36360, XP002316406 ISSN: 0021-9258
- YOSHIZUMI TAKESHI ET AL: "An Arabidopsis cell cycle-dependent kinase-related gene, CDC2b, plays a role in regulating seedling growth in darkness" PLANT CELL, vol. 11, no. 10, October 1999 (1999-10), pages 1883-1895, XP002316407 ISSN: 1040-4651
- HASHIMOTO J ET AL: "Isolation and characterization of cDNA clones encoding cdc2 homologues from Oryza sativa: a functional homologue and cognate variants" MOLECULAR AND GENERAL GENETICS, SPRINGER VERLAG, BERLIN, DE, vol. 233, no. 1/2, May 1992 (1992-05), pages 10-16, XP002113196 ISSN: 0026-8925
- DATABASE EMBL [Online] 9 March 2001 (2001-03-09), "Arabidopsis thaliana mRNA for cyclin dependent kinase (CDKB1;2 gene)" XP002316408 retrieved from EBI accession no. EM_PRO:ATH297937 Database accession no. ATH297937 & DATABASE TREMBL DATABASE [Online] EBI.AC.UK; Putative cell division control CDK 1 May 1999 (1999-05-01), ROUNSLEY, S.D., ET AL.: "Arabidopsis thalianan -Putative cell division control CDK" retrieved from EBI.AC.UK accession no. WWW.EBI.AC.UK Database accession no. Q9ZVI4
- DATABASE EMBL [Online] 17 July 2001 (2001-07-17), "Arabidopsis thaliana Arath;CDKB2;1 mRNA for cyclin-dependent kinase B2, complete cds." XP002316409 retrieved from EBI accession no. EM_PRO:AB047279 Database accession no. AB047279
- MIRONOV V ET AL: "Cyclin-dependent kinases and cell division in plants: The Nexus" PLANT CELL, AMERICAN SOCIETY OF PLANT PHYSIOLOGISTS, ROCKVILLE, MD, US, vol. 11, no. 4, April 1999 (1999-04), pages 509-521, XP002234257 ISSN: 1040-4651
- IMAJUKU Y ET AL: "EXON-INTRON ORGANIZATION OF THE ARABIDOPSIS-THALIANA PROTEIN KINASE GENES CDC2A AND CDC2B" FEBS LETTERS, vol. 304, no. 1, 1992, pages 73-77, XP002268186 ISSN: 0014-5793
- JOUBES J ET AL: "CDK-RELATED PROTEIN KINASES IN PLANTS" PLANT MOLECULAR BIOLOGY, NIJHOFF PUBLISHERS, DORDRECHT, NL, vol. 43, no. 5/6, August 2000 (2000-08), pages 607-620, XP001037063 ISSN: 0167-4412
- HIRAYAMA T ET AL: "IDENTIFICATION OF TWO CELL-CYCLE-CONTROLLING CDC2 GENE HOMOLOGS IN ARABIDOPSIS-THALIANA" GENE (AMSTERDAM), vol. 105, no. 2, 1991, pages 159-166, XP001042260 ISSN: 0378-1119
- MAGYAR Z ET AL: "Cell cycle phase specificity of putative cyclin-dependent kinase variants in synchronized alfalfa cells" PLANT CELL, AMERICAN SOCIETY OF PLANT PHYSIOLOGISTS, ROCKVILLE, MD, US, vol. 9, no. 9, February 1997 (1997-02), pages 223-235, XP002113325 ISSN: 1040-4651 cited in the application

## Description

The present invention relates generally to the field of molecular biology and concerns a method for improving plant growth characteristics. More specifically, the present invention concerns a method for improving plant growth characteristics by increasing expression in a plant of a nucleic acid encoding a B-type CDK (cyclin dependent kinase) protein by introducing and expressing in a plant a genetic construct comprising a B-type CDK nucleic acid encoding a B-type CDK protein comprising: (i) a PPTALRE motif with no mismatches or with a mismatch at position 2 and/or 4 from left to right; (ii) a catalytic kinase domain; and (iii) T-loopactivation domain. The present invention also concerns plants obtained by the methods of the invention, which plants have improved growth characteristics relative to corresponding wild type plants.

The ever-increasing world population and the dwindling supply of arable land available for agriculture fuel agricultural research towards improving the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits. A trait of particular economic interest is yield. Yield is normally defined as the measurable produce of economic value from a crop. This may be defined in terms of quantity and/or quality. Yield is directly dependent on several factors, for example, the number and size of the organs, plant architecture (for example, the number of branches), seed production and more. Root development, nutrient uptake and stress tolerance are also important factors in determining yield. Typical stresses to which plants are subjected include environmental (abiotic) stresses (such as temperature stresses caused by atypical high or low temperatures; stresses caused by nutrient deficiency; stresses caused by lack of water (drought)) and biotic stresses (which can be imposed on plants by other plants (weeds), animal pests and pathogens). Crop yield may be increased not only by optimising one of the abovementioned factors, but may also be increased by modifying the inherent growth mechanisms of a plant.

The inherent growth mechanisms of a plant reside in a highly ordered sequence of events collectively known as the 'cell cycle'. Progression through the cell cycle is fundamental to the growth and development of all multicellular organisms and is crucial to cell proliferation. The major components of the cell cycle are highly conserved in yeast, mammals, and plants. The cell cycle is typically divided into the following sequential phases: G0 - G1 - S - G2 - M. DNA replication or synthesis generally takes place during the S phase ("S" is for DNA synthesis) and mitotic segregation of the chromosomes occurs during the M phase (the "M" is for mitosis), with intervening gap phases, G1 (during which cells grow before DNA replication) and G2 (a period after DNA replication during which the cell prepares for division). Cell division is completed after cytokinesis, the last step of the M phase. Cells that have exited the cell cycle and that have become quiescent are said to be in the G0 phase. Cells in this phase can be stimulated to renter the cell cycle at the G1 phase. The "G" in G1, G2 and G0 stands for "gap". Completion of the cell cycle process allows each daughter cell during cell division to receive a full copy of the parental genome.

Cell division is controlled by two principal cell cycle events, namely initiation of DNA synthesis and initiation of mitosis. Each transition to each of these key events is controlled by a checkpoint represented by specific protein complexes (involved in DNA replication and division). The expression of genes necessary for DNA synthesis at the G1/S boundary is regulated by the E2F family of transcription factors in mammals and plant cells (La Thangue, 1994 (Curr. Opin. Cell Biol. 6 (3), 443 - 450); Muller et al., 2001 (Genes Dev. 15 (3), 267 - 285); De Veylder et al., 2002 (EMBO J. 21 (6), 1360 - 1368)). Entry into the cell cycle is regulated/triggered by an E2F/Rb complex that integrates signals and allows activation of transcription of cell cycle genes. The transition between the different phases of the cell cycle, and therefore progression through the cell cycle, is driven by the formation and activation of different heterodimeric serine/threonine protein kinases, generally referred to as cyclin-dependent kinases (CDKs). A prerequisite for activity of these kinases is the physical association with a specific cyclin, the timing of activation being largely dependent upon cyclin expression. Cyclin binding induces conformational changes in the N-terminal lobe of the associating CDK and contributes to the localisation and substrate specificity of the complex. Monomeric CDKs are activated when they are associated with cyclins and thus have kinase activity. Cyclin protein levels fluctuate in the cell cycle and therefore represent a major factor in determining timing of CDK activation. The periodic activation of these complexes containing cyclins and CDK during cell cycle mediates the temporal regulation of cell-cycle transitions (checkpoints). Other factors regulating CDK activity include CDK inhibitors (CKls or ICKs, KIPs, CIPs, INKS), CDK activating kinases (CAKs), a CDK phosphatase (Cdc25) and a CDK subunit (CKS) (Mironov et al. 1999 (Plant Cell 11 (4), 509 - 522); Reed 1996 (Progressin Cell cycle Research 2, 15 - 27)).

In plants, two major classes of CDKs, known as A-type and B-type CDKs, have been studied to date. The A-type CDKs regulate both the G1-to-S and G2-to-M transitions, whereas the B-type CDKs seem to control the G2-to-M checkpoint only (Hemerly et al., 1995 (EMBO J. 14 (16), 3925 - 3936); Magyar et al., 1997 (Plant Cell 9 (2), 223 - 235); Porceddu et al., 2001 (J. Biol. Chem. 276 (39) 36354 - 36360)). In addition, the presence of C-type CDKs and CDK-activating kinases (CAKs) has been reported (Magyar et al., 1997 (Plant Cell 9 (2), 223 - 235); Umeda et al., 1998 (Proc Natl Acad Sci USA. 95 (9), 5021 - 5026.; Joubès et al., 2001 (Plant Physiol. 126 (4), 1403 - 1415)), as has the presence of D-type, E-type and F-type CDKs (Vandepoele et al. 2002 (Plant Cell 14 (4), 903 - 916)).

The ability to influence the cell cycle in a plant, and to thereby modify various growth characteristics of a plant, would have many applications in areas such as crop enhancement, plant breeding, production of ornamental plants, aboriculture, horticulture, forestry, the production of algae or plants (for example for use as bioreactors, for the production of substances such as pharmaceuticals, antibodies, or vaccines, or for the bioconversion of organic waste or for use as fuel in the case of high-yielding algae and plants).

It has now been found that increasing expression in a plant of a B-type CDK nucleic acid encoding a B-type CDK protein comprising; (i) a PPTALRE motif with no mismatches or with a mismatch at position 2 and/or 4 from left to right; (ii) a catalytic kinase domain; and (iii) T-loop activation kinase domain, gives plants having improved growth characteristics. Therefore according to a first embodiment of the present invention there is provided a method for (improving) the growth characteristics of a plant, comprising increasing expression in a plant of a B-type CDK nucleic acid, encoding a B-type CDK protein comprising: (i) a PPTALRE motif with no mismatches or with a mismatch at position 2 and/or 4 from left to right; (ii) a catalytic kinase domain; and (iii) T-loop activation kinase domain, , wherein the improved growth characteristics are selected from one or more of: an increase in area, an increase in the number of panicles, an increase in height, an increase in the number of seeds, an increase in the number of filled seeds, an increase in the total weight of seeds, an increase in thousand kernel weight (TKW) and an increase in harvest index. Increasing expression of a B-type CDK nucleic acid is enhancing or increasing expression of a B-type CDK gene/nucleic acid. The increased expression, is increased compared to expression, of a B-type CDK in corresponding wild-type plants. Methods for obtaining enhanced or increased expression of genes or gene products are well documented in the art and include, for example, overexpression driven by a strong promoter, the use of transcription enhancers or translation enhancers.

The method for improving) the growth characteristics of plants comprises introducing and expressing in a plant a B-type CDK nucleic acid, encoding a B-type CDK protein comprising: (i) a PPTALRE motif with no mismatches or with a mismatch at position 2 and/or 4 from left to right; (ii) a catalytic kinase domain; and (iii) T-loop activation kinase domain. The nucleic acid may be introduced into a plant by, for example, transformation. Therefore, according to a preferred aspect of the present invention, there is provided a method for improving the growth characteristics of a plant comprising introducing into a plant, in an expressible format, a B-type CDK nucleic acid, encoding a B-type CDK protein comprising; (i) a PPTALRE motif with no mismatches or with a mismatch at position 2 and/or 4 from left to right; (ii) a catalytic kinase domain: and (iii) T-loop activation kinase domain, , wherein the improved growth characteristics are one or more of: an increase in area, an increase in the number of panicles, an increase in height, an increase in the number of seeds, an increase in the number of filled seeds, an increase in the total weight of seeds, an increase in thousand kernel weight (TKW) and an increase in harvest index, each relative to corresponding wild- type plants.

A "B-type CDK nucleic acid" as defined herein is a nucleic acid/gene encoding a protein having: (i) a PPTALRE motif with no mismatches or with a mismatch at position 2 and/or 4 from left to right; (ii) a catalytic kinase domain; and (iii) a T-loop activation kinase domain (Magyar et al., 1997 (Plant Cell 9 (2), 223 - 235)).

The motifs and domains identified in (i) to (iii) above may easily be identified by persons skilled in the art using routine techniques. A kinase assay may, for example, be performed as described in Cockcroft et al., 2000 (Nature, 405 (6786), 575-579). The assay involves grinding plant material in liquid nitrogen and resuspending in an appropriate buffer (such as 1 ml of 50mM Tris-HCl pH 7.5, 75mM NaCl, 15mM EGTA, 15mMMgCl2, 1 mM dithiothreitol, 0.1% Tween 20, 1X complete Tm protease inhibitor, 1 mMNaF, 0.2mM NaV, 2mM Na-pyrophosphate, 60mM beta-glycerophosphate). The suspension is then homogenized for 4 x 30sec with 30 sec on ice between homogeneizations. The supernatant is then incubated with 20 microliters of protein A Sepharose (50% suspension) for 30 min at 4°C. The supernatant is incubated with 1 microliter of antiserum (directed against the carboxy terminal peptide of a CDC2b protein, as described in Setiady et al., 1996 (Plant Cell Physiology 37 (3), 369-376) for 2h on ice, then 20 microliters protein A-Sepharose added and the sample rotated at 4°C for an hour. Samples are washed 2 times with kinase buffer (50mM Tris-HCl pH7.5, 100mM NaCl, 5mM EGTA, 1mM DTT), resuspended in 15 microliters assay buffer (50mM Tris-HCl pH7.5, 100mM NaCl, 5mM EGTA, 10mM MgCl2, 1mM DTT, 1 mM NaF, 0.2 mM Na-Vanadate, 2 mM Na pyrophosphate, 25mM beta glycerophosphate, 0.5 mM histone H1 as a substrate, 0.5mM PMSF, and 74 kBq [gamma ³²P ATP (>185 TBq mmol-1) per 15 microliters of reaction) and incubated at room temperature for 30 min. The reaction may be stopped by adding gel loading buffer and samples may be analysed using SDS-PAGE and quantified using a phosphoimager (Molecular Dynamics).

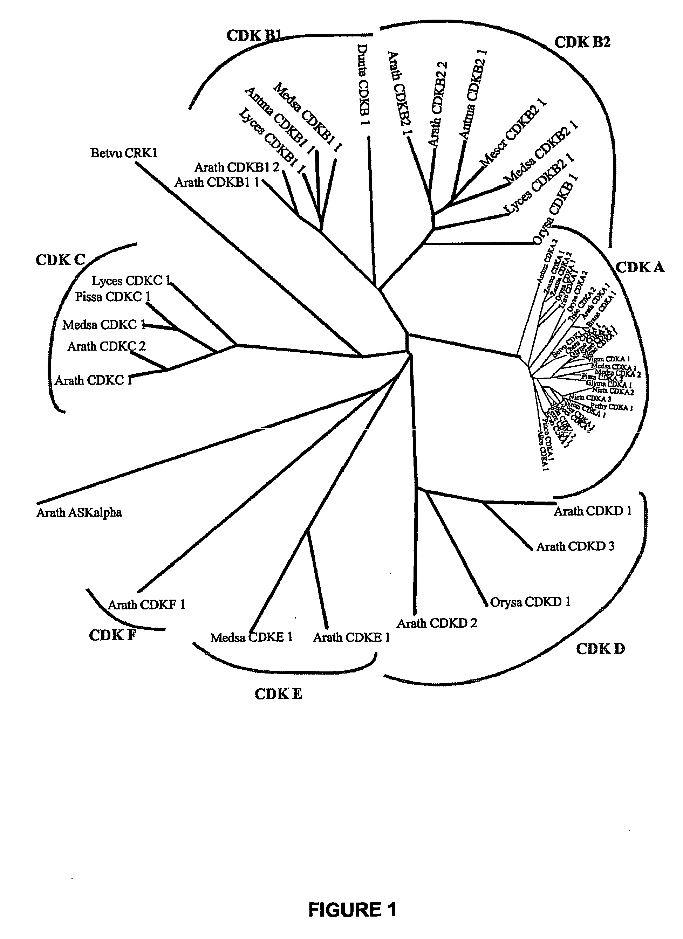

The term "B-type CDK amino acid" as defined herein encompasses any amino acid sequence which when used in the construction of a CDK phylogenetic tree, such as the one depicted in Fig. 1, tends to cluster around the B-type CDKs rather than any of the other CDK groups. A person skilled in the art could readily determine whether any amino acid sequence in question falls within the definition of a "B-type CDK amino acid" using known techniques and software for the making of such a phylogenetic tree, such as a GCG, EBI or CLUSTAL package, using default parameters. Upon construction of such a phylogenetic tree, sequences clustering in the B-type CDK group will be considered to fall within the definition of a "B-type CDK" and will therefore be useful in performing the methods of the invention. Additionally, a "B-type CDK amino acid" as defined herein is one comprising: (i) a PPTALRE motif with no mismatches or with a mismatch at position 2 and/or 4 from left to right; (ii) a catalytic kinase domain; (iii) a T-loop activation kinase domain (Magyar et al., 1997 (Plant Cell 9 (2), 223 - 235)).

Table 1 below shows representative B-type CDKs and the PPTALRE motif with no mismatches or with a mismatch at position 2 and/or 4 from left to right.

**Table 1**

| **B-type CDK** | **NCBI Accession Number** | **Motif** |
|---|---|---|
| *Arabidopsis* CDKB1.1 | At3g54180 | PPTALRE |
| *Arabidopsis* CDKB1.2 | At2g38620 | PPTALRE |
| Tobacco CDK31-1 | AF289465 | PPTALRE |
| Tobacco CDKB1-2 | AF289466 | PPTALRE |
| *Medicago* CDC2MsD | X97315 | PPTALRE |
| Tomato CDKB1 | AJ297916 | PPTALRE |
| Sunflower CDKB1.1 | AY063463 | PPTALRE |
| *Antirrhinum* CDC2 kinase | X97639 | PPTALRE |
| *Chenopodium* CDK | AJ278885 | PPTALRE |
| *Oryza* CDK predicted | NM_190272 | PPTALRE |
| *Oryza* CDK predicted | D64036 | PPTALRE |
| Maize CDK predicted | AY106440 | PPTALRE |
| Maize CDK predicted | AY106029 | PPTALRE |
| Wheat CDK predicted | BT009182 | PPTALRE |
| *Medicago* CDC2MSF | X97317 | PPTTLRE |
| Populus CDKB | AY307372 | PPTTLRE |
| Tomato CDKB2 | AJ297917 | PPTTLRE |
| Soybean CDKB | AY439096 | PPTTLRE |
| *Antirrhinum* CDC2 kinase | X97640 | PPTTLRE |
| *Arabidopsis* CDKB2.2 | At1g20930 | PPTTLRE |
| *Arabidopsis* CDKB2.1 | At1g76540 | PSTTLRE |

The B-type CDK nucleic acid/gene may be isolated or derived from any plant or algal or fungal source. This nucleic acid may be substantially modified from its native form in composition and/or genomic environment through deliberate human manipulation. The B-type CDK nucleic acid may be isolated from a monocotyledonous or dicotyledonous species, preferably from the family *Brassicaceae,* further preferably from *Arabidopsis thaliana.* The nucleic acid is preferably a class 1 B-type CDK, such as a class 1 B-type CDK selected from the examples of class 1 CDKs shown in Fig. 1, namely, CDK B1;1 from *Arabidopsis thaliana,* CDK B1;2 from *Arabidopsis thaliana,* CDKB1;1 from *Lycopersicon esculentum* (tomato), CDK 81;1 from *Antirrhinum majus,* CDK B1;1 from *Medicago sativa* (alfalfa) and CDK B1 from *Dunaliella tertiolecta,* further preferably the class 1 B-type CDK is a CDK B1;1 from *Arabidopsis thaliana* or a CDK B1;2 from *Arabidopsis thaliana.* Alternatively, the nucleic acid is preferably a class 2 B-type CDK, such as a class 2 B-type CDK selected from the examples shown in Fig 1, namely, a CDK B2;1 from *Arabidopsis thaliana,* a CDK B2;2 from *Arabidopsis thaliana,* a CDK B2;1 from *Antirrhinum majus,* a CDK B2;1 from *Mesembryanthemum crassifolium,* a CDK B2;1 1 from *Medicago sativa,* a CDK B2;1 from *Lycopersicon esculentum* and a CDK B 1 from *Oryza sativa,* further preferably the class 2 B-type CDK is a CDK B2;2 from *Arabidopsis thaliana.*

Most preferably the CDK B1;1 nucleic acid is as represented by SEQ ID NO: 1 or by a portion thereof, or by a nucleic acid sequence capable of hybridising therewith, and wherein the CDK B1;1 protein is as represented by SEQ ID NO: 2, or a homologue, derivative or active fragment thereof. Most preferably the CDK B1;2 nucleic acid is as represented by SEQ ID NO: 3 or by a portion thereof, or by a nucleic acid sequence capable of hybridising therewith, and wherein the CDK B1;2 protein is as represented by SEQ ID NO: 4, or a homologue, derivative or active fragment thereof. Most preferably the CDK B2;2 nucleic acid is as represented by SEQ ID NO: 5 or by a portion thereof, or by a nucleic acid sequence capable of hybridising therewith, and wherein the CDK B2;2 protein is as represented by SEQ ID NO: 6, or a homologue, derivative or active fragment thereof. Each of the CDK B1;1, CDKB1;2 and CDK B2;2 nucleic acids/proteins also encompass the variant nucleic acids and amino acids as described hereinafter.

Although the invention has been exemplified with a B-type CDK according to SEQ ID NO: 1, SEQ ID NO: 3 and SEQ ID NO: 5, and corresponding amino acids according to SEQ ID NO: 2, SEQ ID NO: 4 and SEQ ID NO: 6, respectively, it would be apparent to a person skilled in the art that the methods according to the invention may also be practised using variant nucleic acids and variant amino acids, such as the ones defined hereinafter. Therefore, taken in a broad context, the term "B-type CDK" protein/nucleic acid also encompasses variant nucleic acids and variant amino acids suitable for practising the methods according to the invention. Variant nucleic acids and variant amino acids suitable for practising the methods according to the invention include those falling within the definition of a "B-type CDK", meaning that upon construction of a phylogenetic tree, such as the one depicted in Fig. 1, the variant sequences of interest would tend to cluster around the B-type CDKs and/or which variant encodes (in the case of a variant nucleic acid) and is (in the case of a variant amino acid) a protein I comprising: (i) a PPTALRE motif with no mismatches or with a mismatch at position 2 and/or 4 from left to right; (ii) a catalytic kinase domain; (iii) a T-loop activation kinase domain (Magyar et al., 1997 (Plant Cell 9 (2), 223 - 235)).

Suitable variant nucleic acid and amino acid sequences useful in practising the method according to the invention, include:
(i) Functional portions of a B-type CDK nucleic acid/gene;
(ii) Sequences capable of hybridising with a B-type CDK nucleic acid/gene;
(iii) Alternative splice variants of a B-type CDK nucleic acid/gene;
(iv) Allelic variants of a B-type CDK nucleic acid/gene;
(v) Homologues, derivatives and active fragments of a B-type CDK protein;
(vi) Mutant B-type CDKs.

An example of a variant B-type nucleic acid/gene is a functional portion of a B-type nucleic acid/gene. It would be apparent to a person skilled in the art that the full length DNA sequence is not a prerequisite to carrying out the methods according to the invention. The methods according to the invention may advantageously be practised using functional portions of a B-type CDK. A functional portion refers to a piece of DNA derived or prepared from an original (larger) B-type CDK DNA molecule, which DNA portion, when introduced and expressed in a plant, gives plants having modified growth characteristics, which portion encodes a protein comprising: (i) a PPTALRE motif with no mismatches or with a mismatch at position 2 and/or 4 from left to right; (ii) a catalytic kinase domain; (iii) a T-loop activation kinase domain (Magyar et al., 1997 (Plant Cell 9 (2), 223 - 235)). The portion may comprise many genes, with or without additional control elements or may contain spacer sequences. The portion may be made by making one or more deletions and/or truncations to the nucleic acid sequence of, for example, any one of SEQ ID NO: 1, SEQ ID NO: 3 and SEQ ID NO: 5. Techniques for introducing truncations and deletions into a nucleic acid are well known in the art.

An example of a further variant B-type CDK nucleic acid is a sequence that is capable of hybridising to a B-type CDK. Advantageously, the methods according to the present invention may also be practised using sequences capable of hybridising to a B-type CDK, particularly a B-type CDK as represented by any one of SEQ ID NO: 1, SEQ ID NO: 3 and SEQ ID NO: 5, which hybridising sequences are those falling within the definition of a "B-type CDK", meaning that upon construction of a phylogenetic tree, such as the one depicted in Fig. 1, the hybridising sequence would be one that tends to cluster around the B-type CDKs rather than any of the other CDK groups and/of which hybridising sequence encodes a protein comprising: I (i) a PPTALRE motif with no mismatches or with a mismatch at position 2 and/or 4 from left to right; (ii) a catalytic kinase domain; (iii) a T-loop activation kinase domain (Magyar et al., 1997 (Plant Cell 9 (2), 223 - 235)).

The term "hybridisation" as defined herein is a process wherein substantially homologous complementary nucleotide sequences anneal to each other. The hybridisation process can occur entirely in solution, i.e. both complementary nucleic acids are in solution. Tools in molecular biology relying on such a process include the polymerase chain reaction (PCR; and all methods based thereon), subtractive hybridisation, random primer extension, nuclease S1 mapping, primer extension, reverse transcription, cDNA synthesis, differential display of RNAs, and DNA sequence determination. The hybridisation process can also occur with one of the complementary nucleic acids immobilised to a matrix such as magnetic beads, Sepharose beads or any other resin. Tools in molecular biology relying on such a process include the isolation of poly (A+) mRNA. The hybridisation process can furthermore occur with one of the complementary nucleic acids immobilised to a solid support such as a nitro-cellulose or nylon membrane or immobilised by e.g. photolithography to e.g. a siliceous glass support (the latter known as nucleic acid arrays or microarrays or as nucleic acid chips). Tools in molecular biology relying on such a process include RNA and DNA gel blot analysis, colony hybridisation, plaque hybridisation, *in situ* hybridisation and microarray hybridisation. In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration and hybridisation buffer composition. High stringency conditions for hybridisation include high temperature and/or low salt concentration (salts include NaCl and Na₃-citrate) and/or the inclusion of formamide in the hybridisation buffer and/or lowering the concentration of compounds such as SDS (detergent) in the hybridisation buffer and/or exclusion of compounds such as dextran sulphate or polyethylene glycol (promoting molecular crowding) from the hybridisation buffer. Conventional hybridisation conditions are described in, for example, Sambrook (2001) Molecular Cloning: a laboratory manual, 3rd Edition Cold Spring Harbor Laboratory Press, CSH, New York, but the skilled craftsman will appreciate that numerous different hybridisation conditions can be designed in function of the known or the expected homology and/or length of the nucleic acid sequence. Sufficiently low stringency hybridisation conditions are particularly preferred (at least in the first instance) to isolate nucleic acids heterologous to the DNA sequences of the invention defined supra. An example of low stringency conditions is 4-6x SSC / 0.1-0.5% w/v SDS at 37-45°C for 2-3 hours. Depending on the source and concentration of the nucleic acid involved in the hybridisation, alternative conditions of stringency may be employed, such as medium stringency conditions. Examples of medium stringency conditions include 1-4x SSC / 0.25% w/v SDS at ≥ 45°C for 2-3 hours. An example of high stringency conditions includes 0.1-1x SSC / 0.1% w/v SDS at 60°C for 1-3 hours. The skilled man will be aware of various parameters which may be altered during hybridisation and washing and which will either maintain or change the stringency conditions. The stringency conditions may start low and be progressively increased until there is provided a hybridising B-type CDK nucleic acid, as defined hereinabove. Elements contributing to heterology include allelism, degeneration of the genetic code and differences in preferred codon usage.

Another example of a variant B-type CDK is an alternative splice variant of a B-type CDK. The methods according to the present invention may also be practised using an alternative splice variant of a 3-type CDK nucleic acid/gene. The term "alternative splice variant" was used herein encompasses variants of a nucleic acid in which selected introns and/or exons have been excised, replaced or added. Such splice variants may be found in nature or can be manmade using techniques well known in the art. The splice variants useful in the methods according to the invention are "B-type CDKs", meaning that upon construction of a phylogenetic tree, such as the one depicted in Fig. 1, the splice variant of interest would be one tending to cluster around the B-type CDKs rather than around any of the other CDK groups and/of which splice I variant encodes a protein comprising: (i) a PPTALRE motif with no mismatches or with a mismatch at position 2 and/or 4 from left to right; (ii) a catalytic kinase domain; (iii) a T-loop activation kinase domain (Magyar et al., 1997(Plant Cell 9 (2), 223 - 235)). Preferably, the splice variant is a splice variant of the sequence represented by any of SEQ ID NO: 1, SEQ ID NO: 3 and SEQ ID NO: 5

Another example of a variant B-type CDK is an allelic variant. Advantageously, the methods according to the present invention may also be practised using allelic variants of a B-type CDK nucleic acid, preferably an allelic variant of a sequence represented by any of SEQ ID NO: 1, SEQ ID NO: 3 and SEQ ID NO: 5. Allelic variants exist in nature and encompassed within the methods of the present invention is the use of these isolated natural alleles in the methods according to the invention. The allelic variants useful in the methods according to the invention are "B-type CDKs", meaning that upon construction of a phylogenetic tree, such as the one depicted in Fig. 1, the allelic variant of interest would tend to cluster around the B-type CDKs and which allelic variant encodes a protein comprising: (i) a PPTALRE motif with no mismatches or with a mismatch at position 2 and/or 4 from left to right; (ii) a catalytic kinase domain; (iii) a T-loop activation kinase domain (Magyar et al., 1997 (Plant Cell 9 (2), 223 - 235)).

Examples of variant B-type amino acids include homologues, derivatives and active fragments of a B-type CDK protein. Advantageously, the methods according to the present invention may also be practised using homologues, derivatives or active fragments of a B-type CDK, preferably using nucleic acids encoding homologues, derivatives or active fragments of a B-type CDK as represented by any one of SEQ ID NO: 2, SEQ ID NO: 4 and SEQ ID NO: 6.

"Homologues" of a B-type CDK protein encompass peptides, oligopeptides, polypeptides, proteins and enzymes having amino acid substitutions, deletions and/or insertions relative to the unmodified protein in question and having similar biological and functional activity as the unmodified protein from which they are derived. To produce such homologues, amino acids of the protein may be replaced by other amino acids having similar properties (such as similar hydrophobicity, hydrophilicity, antigenicity, propensity to form or break α-helical structures or β-sheet structures). Conservative substitution tables are well known in the art (see for example Creighton (1984) Proteins. W.H. Freeman and Company). The homologues useful in the methods according to the invention are preferably B-type CDKs, meaning that upon construction of a phylogenetic tree, such as the one depicted in Fig. 1, any homologous sequences of interest would tend to cluster around B-type CDKs rather than any other group of CDKs. Such B-type CDKs have in increasing order of preference at least 60% or 65% or 70% or 75% or 80% or 85% or 90% or 95%, 96%, 97%, 98%, 99% or more, sequence identity or similarity to that of the *Arabidopsis thaliana* CDK B1;1 (SEQ ID NO: 2) and which homologue comprises: (i) a PPTALRE motif with no mismatches or with a mismatch at position 2 and/or 4 from left to right; (ii) a catalytic kinase domain; (iii) a T-loop activation kinase domain (Magyar et al., 1997 (Plant Cell 9 (2), 223 - 235)).

Whether a polypeptide has at least 60% identity to the *Arabidopsis thaliana* CDK B1;1 may readily be established by sequence alignment. Methods for the alignment of sequences for comparison are well known in the art, such methods include GAP, BESTFIT, BLAST, FASTA and TFASTA. GAP uses the algorithm of Needleman and Wunsch 1970 (J. Mol. Biol. 48 (3), 443 - 453) to find the alignment of two complete sequences that maximises the number of matches and minimises the number of gaps. The BLAST algorithm calculates percent sequence identity and performs a statistical analysis of the similarity between the two sequences. The software for performing BLAST analysis is publicly available through the National Centre for Biotechnology Information. A protein having at least 60% identity to the *Arabidopsis thaliana* CDK B1;1 may readily be identified by aligning a query sequence with known B-type CDK sequences (see Fig. 1 for example) using, for example, the VNTI AlignX multiple alignment program, based on a modified clustal W algorithm (InforMax, Bethesda, MD, http://www.informaxinc.com), with default settings for gap opening penalty of 10 and a gap extension of 0.05.

Two special forms of homology: orthologs and paralogs, are evolutionary concepts used to describe the ancestral relationships of genes. The term "paralogous" relates to gene-duplications within the genome of a species leading to paralogous genes. The term "orthologous" relates to homologous genes in different organisms due to ancestral relationship. The term "homologues" as used herein also encompasses paralogues and orthologues of the proteins useful in the methods according to the invention.

Othologues in, for example, monocot plant species may easily be found by performing a so-called reciprocal blast search. This may be done by a first blast involving blasting the sequence in question (for example, SEQ ID NO: 1 or SEQ ID NO- 2) against any sequence database, such as the publicly available NCBI database which may be found at: http://www.ncbi.nlm.nih.gov. If orthologues in rice were sought, the sequence in question would be blasted against, for example, the 28,469 full-length cDNA clones from *Oryza sativa* Nipponbare available at NCBI. BLASTn or tBLASTX may be used when starting from nucleotides or BLASTP or TBLASTN when starting from the protein, with standard default values. The blast results may be filtered. The full-length sequences of either the filtered results or the non-filtered results are then blasted back (second blast) against the sequences of the organism from which the sequence in question is derived. The results of the first and second blasts are then compared. An orthologue is found when the results of the second blast give as hits with the highest similarity a B-type CDK nucleic acid or protein, for example, if one of the organisms is *Arabidopsis* then a paralogue is found. In the case of large families, ClustalW may be used, followed by a neighbour joining tree, to help visualize the clustering.

"Substitutional variants" of a protein are those in which at least one residue in an amino acid sequence has been removed and a different residue inserted in its place. Amino acid substitutions are typically of single residues, but may be clustered depending upon functional constraints placed upon the polypeptide; insertions will usually be of the order of about 1 to 10 amino acid residues and deletions will range from about 1 to 20 residues. Preferably, amino acid substitutions comprise conservative amino acid substitutions. Substitutional variants useful in the methods of the invention will be those comprising: (i) a PPTALRE motif with no mismatches or with a mismatch at position 2 and/or 4 from left to right; (ii) a catalytic kinase domain; and (iii) a T-loop activation kinase domain (Magyar et al., 1997 (Plant Cell 9 (2), 223 - 235)).

"Insertional variants" of a protein are those in which one or more amino acid residues are introduced into a predetermined site in a protein. Insertions can comprise amino-terminal and/or carboxy-terminal fusions as well as intra-sequence insertions of single or multiple amino acids. Generally, insertions within the amino acid sequence will be smaller than amino- or carboxy-terminal fusions, of the order of about 1 to 10 residues. Examples of amino- or carboxy-terminal fusion proteins or peptides include the binding domain or activation domain of a transcriptional activator as used in the yeast two-hybrid system, phage coat proteins, (histidine)6-tag, glutathione S-transferase-tag, protein A, maltose-binding protein, dihydrofolate reductase, Tag·100 epitope, c-myc epitope, FLAG®-epitope, lacZ, CMP (calmodulin-binding peptide), HA epitope, protein C epitope and VSV epitope. Insertional variants useful in the methods of the invention will be those comprising: (i) a PPTALRE motif with no mismatches or with a mismatch at position 2 and/or 4 from left to right; (iii) a T-loop activation kinase domain (Magyar et al., 1997 (Plant Cell 9 (2), 223 - 235)).

"Deletion variants" of a protein are characterised by the removal of one or more amino acids from the protein. Amino acid variants of a protein may readily be made using peptide synthetic techniques well known in the art, such as solid phase peptide synthesis and the like, or by recombinant DNA manipulations. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen *in vitro* mutagenesis (USB, Cleveland, OH), QuickChange Site Directed mutagenesis (Stratagene, San Diego, CA), PCR-mediated site-directed mutagenesis or other site-directed mutagenesis protocols. Deletional variants useful in the methods of the invention will be those comprising: (i) a PPTALRE motif with no mismatches or with a mismatch at position 2 and/or 4 from left to right; (ii) a catalytic kinase domain; (iii) a T-loop activation kinase domain (Magyar et al., 1997 (Plant Cell 9 (2), 223 - 235)).

Methods for the search and identification of B-type CDK homologues would be well within the realm of a person skilled in the art. Methods for the alignment of sequences for comparison

are well known in the art. Such methods include GAP, BESTFIT, BLAST, FASTA and TFASTA. GAP uses the algorithm of Needleman and Wunsch 1970 (J. Mol. Biol. 48 (3), 443 - 453) to find the alignment of two complete sequences that maximises the number of matches and minimises the number of gaps. The BLAST algorithm calculates percent sequence identity and performs a statistical analysis of the similarity between the two sequences. The software for performing BLAST analysis is publicly available through the National Centre for Biotechnology Information.

The term "derivatives" refers to peptides, oligopeptides, polypeptides, proteins and enzymes which may comprise substitutions, deletions or additions of naturally and non-naturally occurring amino acid residues compared to the amino acid sequence of a naturally-occurring form of the protein, for example, as represented by any one of SEQ ID NO: 2, SEQ ID NO: 4 and SEQ ID NO: 6. "Derivatives" of a B-type CDK protein encompass peptides, oligopeptides, polypeptides, proteins and enzymes which may comprise naturally occurring altered, glycosylated, acylated or non-naturally occurring amino acid residues compared to the amino acid sequence of a naturally-occurring form of the polypeptide. A derivative may also comprise one or more non-amino acid substituents compared to the amino acid sequence from which it is derived, for example a reporter molecule or other ligand, covalently or non-covalently bound to the amino acid sequence such as, for example, a reporter molecule which is bound to facilitate its detection, and non-naturally occurring amino acid residues relative to the amino acid sequence of a naturally-occurring protein. Derivatives useful in the methods of the invention will be those comprising: (i) a PPTALRE motif with no mismatches or with a mismatch at position 2 and/or 4 from left to right; (ii) a catalytic kinase domain; (iii) a T-loop activation kinase domain (Magyar et al., 1997 (Plant Cell 9 (2), 223 - 235)).

"Active fragments" of a B-type CDK protein comprise: (i) a PPTALRE motif with no mismatches or with a mismatch at position 2 and/or 4 from left to right; (ii) a catalytic kinase domain; (iii) a T-loop activation kinase domain (Magyar et al., 1997 (Plant Cell 9 (2), 223 - 235)).

Further advantageously, the methods according to the present invention may also be practised using mutant plant CDKs, which mutant CDKs have at least a substantially similar, preferably enhanced, biological activity compared with corresponding wild-type CDK proteins.

According to a fifth embodiment of the present invention, genetic constructs and vectors to facilitate introduction and/or expression of the nucleotide sequences useful in the methods according to the invention are provided. Therefore, according to a fifth embodiment of the present invention, there is provided a gene construct comprising:
(i) a B-type CDK gene/nucleic acid encoding a B-type CDK protein comprising; (i) a PPTALRE motif with no mismatches or with a mismatch at position 2 and/or 4 from left to right: (ii) a catalytic kinase domain: and (iii) T-loop activation kinase domain; or
(ii) one or more control sequences capable of driving expression of the nucleic acid of (i), said control sequence comprising a constitutive GOS2 promoter or a beta-expansin promoter; and optionally
(iii) a transcription termination sequence.
   Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells.

The nucleic acid encoding a CDK mutant may be any.of the mutant-encoding nucleic acids described hereinbefore.

The B-type CDK gene/nucleic acid may be isolated from a monocotyledonous or dicotyledonous species, preferably from the family *Brassicaceae,* further preferably from *Arabidopsis thaliana.* The nucleic acid is preferably a class 1 B-type CDK, such as a class 1 B-type CDK selected from the examples of class 1 CDKs shown in Fig. 1, namely, CDK B1;1 from *Arabidopsis thaliana,* CDK B1;2 from *Arabidopsis thaliana,* CDKB1;1 from *Lycopersicon esculentum (tomato),* CDK B1;1 from *Antirrhinum majus,* CDK B1;1 from *Medicago sativa* (alfalfa) and CDK B1 from *Dunaliella tertiolecta.* Further preferably the class 1 B-type CDK is a CDK B1;1 from *Arabidopsis thaliana* or a CDK B1;2 from *Arabidopsis thaliana.* Alternatively, the nucleic acid is preferably a class 2 B-type CDK, such as a class 2 B-type CDK selected from the examples shown in Fig 1, namely, a CDK B2;1 from *Arabidopsis thaliana,* a CDK B2;2 from *Arabidopsis thaliana,* a CDK B2;1 from *Antirrhinum majus,* a CDK B2;1 from *Mesembryanthemum crassifolium,* a CDK B2;1 from *Medicago sativa,* a CDK B2;1 from *Lycopersicon esculentum* and a CDK B 1 from *Oryza sativa.* Further preferably the class 2 B-type CDK is a CDK B2;2 from *Arabidopsis thaliana.*

Most preferably the CDK B1;1 nucleic acid is as represented by SEQ ID NO: 1 or by a portion thereof, or by a nucleic acid sequence capable of hybridising therewith, and wherein the CDK B1;1 protein is as represented by SEQ ID NO: 2, or a homologue, derivative or active fragment thereof. Most preferably the CDK B1;2 nucleic acid is as represented by SEQ ID NO: 3 or by a portion thereof, or by a nucleic acid sequence capable of hybridising therewith, and wherein the CDK B1;2 protein is as represented by SEQ ID NO: 4, or a homologue, derivative or active fragment thereof. Most preferably the CDK B2;2 nucleic acid is as represented by SEQ ID NO: 5 or by a portion thereof, or by a nucleic acid sequence capable of hybridising therewith, and wherein the CDK B2;2 protein is as represented by SEQ ID NO: 6, or a homologue, derivative or active fragment thereof. Each of the CDK B1;1, CDKB1;2 and CDK B2;2 nucleic acids/proteins also encompass the variant nucleic acids and amino acids as described hereinbefore.

Plants are then transformed with a construct or vector comprising the sequence of interest (i.e., a B-type CDK nucleic acid encoding a B-type CDK protein or a nucleic acid encoding a CDK mutant), which sequence is operably linked to one or more control sequences (at least a promoter).

The terms "regulatory element", "control sequence" and "promoter" are all used interchangeably herein and are taken to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative which confers, activates or enhances expression of a nucleic acid molecule in a cell, tissue or organ. The terms "control sequence", "regulatory sequence", "regulatory element" and "promoter" are used interchangeably herein. The term "operably linked" as used herein refers to a functional linkage between the promoter sequence and the gene of interest, such that the promoter sequence is able to initiate transcription of the gene of interest.

Advantageously, the B-type CDK nucleic acid may be operably linked to any promoter. Preferably, in the case of a CDK B1;1, expression is driven by a promoter active in young, expanding tissue, such as young leaves, flowers, stems and roots. Such a "young expanding tissue-preferred promoter" as defined herein refers to a promoter that is expressed predominantly in young expanding tissue, but not necessarily exclusively in such tissue. Preferably, the "young expanding tissue-preferred promoter" is the beta-expansin EXPB8 promoter from rice. Other suitable promoters include any expansin promoter, pLEAFY and others. Preferably in the case of a CDK B1;2 and CDK B2;2 expression is driven in a constitutive manner, most preferably wherein the constitutive promoter is a GOS2 promoter. A constitutive promoter is transcriptionally active during most, but not necessarily all, phases of its growth and development. Examples of constitutive plant promoters are given in Table C as shown below. The promoters shown in Table C below may advantageously be used to practise the methods according to the invention.

**Table C: Examples of Constitutive Promoters**

| **Gene Source** | **Expression Pattern** | **Reference** |
|---|---|---|
| Actin | Constitutive | McElroy *et al.,* Plant Cell, 2: 163-171, 1990 |
| CAMV 35S | Constitutive | Odell *et al.,* Nature, 313: 810-812, 1985 |
| CaMV 19S | Constitutive | Nilsson *et al., Physiol. Plant.* 100.456-462, 1997 |
| GOS2 | Constitutive | de Pater *et al*., Plant J Nov;2(6):837-44, 1992 |
| Ubiquitin | Constitutive | Christensen *et al.,* Plant Mol. Biol. 18: 675-689, 1992 |
| Rice cyclophilin | Constitutive | Buchholz *et al.,* Plant Mol Biol. 25(5): 837-43, 1994 |
| Maize H3 histone | Constitutive | Lepetit *et al.,* Mol. Gen. Genet. 231:276-285, 1992 |
| Actin 2 | Constitutive | An *et al.,* Plant J. 10(1); 107-121, 1996 |

Inducible promoters are promoters that have induced or increased transcription initiation in response to a developmental, chemical, environmental or physical stimulus. For example stress-inducible promoters are activated when a plant is exposed to various stress conditions. Examples of stress-inducible promoters, which are also suitable to practise the methods according to the invention, are given in Table D as shown below. Such promoters may also be useful in practising the methods of the invention since modified growth (such as increased growth) induced in times of stress may have many advantages.

**Table D: Examples of Stress-Inducible Promoters**

| **Name** | **Stress** | **Reference** |
|---|---|---|
| P5CS (delta(1)-pyrroline-5-carboxylate syntase) | Salt, water | Zhang *et al.;* Plant Science. Oct 281997; 129(1): 81-89 |
| corl 5a | Cold | Hajela *et al.,* Plant Physiol. 93: 1246-1252 (1990) |
| cor15b | Cold | Wlihelm et al., Plant Mol Biol. 1993 Dec; 23(5):1073-7 |
| cor15a (-305 to +78 nt) | Cold, drought | Baker et al., Plant Mol Biol. 1994 Mar; 24(5): 701-13 |
| rd29 | Salt, drought, cold | Kasuga et al., Nature Biotechnology, vol 18, 287-291, 1999 |
| Heat shock proteins, including artificial promoters containing the heat shock element (HSE) | Heat | Barros et al., Plant Mol Biol, 19(4): 665-75, 1992. Marrs et al., Dev Genet., 14(1): 27-41, 1993. Schoffl etal., Mol Gen Gent, 217(2-3): 246-53, 1989. |
| smHSP (small heat shock proteins) | Heat | Waters et al., J Experimental Botany, vol 47, 296, 325-338, 1996 |
| wcs120 | Cold | Ouellet et al., FEBS Lett. 423, 324-328 (1998) |
| ci7 | Cold | Kirch et al., Plant Mol Biol, 33(5): 897-909, 1997 Mar |
| Adh | Cold, drought, hypoxia | Dolferus et al., Plant Physiol, 105(4): 1075-87, 1994 Aug |
| pwsi18 | Water: salt and drought | Joshee et al., Plant Ceii Physiol, 39(1): 64-72, 1998, Jan |
| ci21 A | Cold | Schneider et al., Plant Physiol, 113(2): 335-45,1997 |
| Trg-31 | Drought | Chaudhary et al., Plant Mol Biol, 30(6): 1247-57, 1996 |
| Osmotin | Osmotic | Raghothama et al., Plant Mol Biol, 23(6): 1117-28,1993 |
| LapA | Wounding, environmental | WO99/03977 University of California/INRA |

The promoters listed in Tables C and D are provided for the purposes of exemplification only and the present invention is not to be limited by the list provided therein. Those skilled in the art will readily be in a position to provide additional promoters that are useful in performing the present invention. The promoters listed may also be modified to provide specificity of expression as required.

Optionally, one or more terminator sequences may also be used in the construct introduced into a plant. The term "terminator" encompasses a control sequence which is a DNA sequence at the end of a transcriptional unit which signals 3' processing and polyadenylation of a primary transcript and termination of transcription. Additional regulatory elements may include transcriptional as well as translational enhancers. Those skilled in the art will be aware of terminator and enhancer sequences which may be suitable for use in performing the invention. Such sequences would be known or may readily be obtained by a person skilled in the art.

The genetic constructs of the invention may further include an origin of replication sequence which is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

The genetic construct may optionally comprise a selectable marker gene. As used herein, the term "selectable marker gene" includes any gene which confers a phenotype on a cell in which it is expressed to facilitate the identification and/or selection of cells which are transfected or transformed with a nucleic acid construct of the invention. Suitable markers may be selected from markers that confer antibiotic or herbicide resistance. Cells containing the recombinant DNA will thus be able to survive in the presence of antibiotic or herbicide concentrations that kill untransformed cells. Examples of selectable marker genes include the bar gene which provides resistance to the herbicide Basta; the npt gene which confers resistance to the antibiotic kanamycin; the hpt gene which confers hygromycin resistance. Visual markers, such as the Green Fluorescent Protein (GFP, Haseloff et al., 1997 (Proc Natl Acad Sci U S A. 94 (6), 2122 - 2127.), β-glucuronidase (GUS) or luciferase may also be used as selectable markers. Further examples of suitable selectable marker genes include the ampicillin resistance (Ampr), tetracycline resistance gene (Tcr), bacterial kanamycin resistance gene (Kanr), phosphinothricin resistance gene, neomycin phosphotransferase gene (nptII), hygromycin resistance gene, gene, and the chloramphenicol acetyltransferase (CAT) gene, amongst others.

The present invention also encompasses plants obtained by the methods according to the present invention. The present invention therefore provides plants obtained by the methods according to the present invention, which plants have improved growth characteristics selected from one or more of: an increase in area, an increase in the number of panicles, an increase in height, an increase in the number of seeds, an increase in the number of filled seeds an increase in the total weight of seeds, an increase in thousand kernel weight (TKW) and an increase in harvest index, and which plants have increased expression of a B-type CDK encoding a B-type CDK protein comprising: (i) a PPTALRE motif with no mismatches or with a mismatch at position 2 and/or 4 from left to right-, (ii) a catalytic kinase domain: and (iii) T-loop activation kinase domain.

The present invention also provides plants having improved growth characteristics selected from one or more of: an increase in area, an increase in the number of panicles, an increase in height, an increase in the number of seeds, an increase in the number of filled seeds, an increase in the total weight of seeds, an increase in thousand kernel weight (TKW) and an increase in harvest index, which plants have increased expression of a B-type CDK encoding a B-type CDK protein comprising; (i) a PPTALRE motif with no mismatches or with a mismatch at position 2 and/or 4 from left to right, (ii) a catalytic kinase domain: and (iii) T-Ioop activation kinase domain.-.

According to a sixth embodiment of the present invention, there is provided a method for the production of transgenic plants having improved growth characteristics selected from one or more of: an increase in area, an increase in the number of panicles, an increase in height, an increase in the number of seeds, an increase in the number of filled seeds, an increase in the total weight of seeds, an increase in thousand kernel weight (TKW) and an increase in harvest index , comprising introduction and expression in a plant of a nucleic acid molecule of the invention.

More specifically, the present invention provides a method for the production of transgenic plants having improved growth characteristics, which method comprises:
(i) introducing and expressing in a plant or a plant cell a B-type CDK gene/nucleic acid encoding a B-type CDK protein comprising; (i) a PPTALRE motif with no mismatches or with a mismatch at position 2 and/or 4 from left to right; (ii) a catalytic kinase domain: and (iii) T-loop activation kinase domain;
(ii) cultivating the plant cell under conditions promoting regeneration and mature plant growth.

The nucleic acid itself may be introduced directly into a plant cell or into the plant itself (including introduction into a tissue, organ or any other part of the plant). According to a preferred feature of the present invention, the nucleic acid is preferably introduced into a plant by transformation.

The B-type CDK nucleic acid/gene is preferably a class 1 B-type CDK, such as a class 1B-type CDK selected from the examples of class 1 CDKs shown in Fig. 1, namely, CDK B1;1 from *Arabidopsis thaliana,* CDK B1;2 from *Arabidopsis thaliana,* CDKB1;1 from *Lycopersicon esculentum (tomato),* CDK B1;1 from *Antirrhinum majus,* CDK B1;1 from *Medicago sativa* (alfalfa) and CDK B1 from *Dunaliella tertiolecta.* Further preferably the class 1 B-type CDK is a CDK B1.1 from *Arabidopsis thaliana* or a CDK B1;2 from *Arabidopsis thaliana.* Alternatively and preferably, the nucleic acid is a class 2 B-type CDK, such as a class 2 B-type CDK selected from the examples shown in Fig 1, namely, a CDK B2;1 from *Arabidopsis thaliana,* a CDK B2;2 from *Arabidopsis thaliana,* a CDK B2;1 from *Antirrhinum majus,* a CDK B2;1 from *Mesembryanthemum crassifolium,* a CDK B2;1 from *Medicago sativa,* a CDK B2;1 from *Lycopersicon esculentum* and a CDK B 1 from Oryza *sativa.* Further preferably the class 2 B-type CDK is a CDK B2;2 from *Arabidopsis thaliana.*

Most preferably the CDK B1;1 nucleic acid is as represented by SEQ ID NO: 1 or by a portion thereof, or by a nucleic acid sequence capable of hybridising therewith, and wherein the CDK B1 ;1 protein is as represented by SEQ ID NO: 2, or a homologue, derivative or active fragment thereof. Most preferably the CDK B1;2 nucleic acid is as represented by SEQ ID NO: 3 or by a portion thereof, or by a nucleic acid sequence capable of hybridising therewith, and wherein the CDK B1;2 protein is as represented by SEQ ID NO: 4, or a homologue, derivative or active fragment thereof. Most preferably the CDK B2;2 nucleic acid is as represented by SEQ ID NO: 5 or by a portion thereof, or by a nucleic acid sequence capable of hybridising therewith, and wherein the CDK B2;2 protein is as represented by SEQ ID NO: 6, or a homologue, derivative or active fragment thereof. Each of the CDK B1;1, CDKB1;2 and CDK B2;2 nucleic acids/proteins also encompass the variant nucleic acids and amino acids as described hereinbefore.

The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell can then be used to regenerate a transformed plant in a manner known to persons skilled in the art.

Transformation of a plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., 1982, Nature 296, 72-74; Negrutiu I. et al., June 1987, Plant Mol. Biol. 8, 363-373); electroporation of protoplasts (Shillito R.D. et al., 1985 Bio/Technol 3, 1099-1102); microinjection into plant material (Crossway A. et al., 1986, Mol. Gen Genet 202, 179-185); DNA or RNA-coated particle bombardment (Klein T.M. et al., 1987, Nature 327, 70) infection with (non-integrative) viruses and the like. Transgenic rice plants expressing a B-type CDK gene are preferably produced via *Agrobacterium*-mediated transformation using any of the well known methods for rice transformation, such as described in any of the following: published European patent application EP 1198985 A1, Aldemita and Hodges (Planta, 199, 612-617, 1996); Chan et al. (Plant Mol. Biol. 22 (3) 491-506, 1993), Hiei et al. (Plant J. 6 (2) 271-282, 1994), which disclosures are incorporated by reference herein as if fully set forth. In the case of corn transformation, the preferred method is as described in either Ishida et al. (Nat. Biotechnol. 1996 Jun; 14(6): 745-50) or Frame et al. (Plant Physiol. 2002 May; 129(1): 13-22), which disclosures are incorporated by reference herein as if fully set forth.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced in the parent by the methods according to the invention. The invention also includes host cells containing an isolated nucleic acid molecule encoding a protein capable of modulating a B-type CDK protein, preferably wherein the protein is a B-type CDK protein. Preferred host cells according to the invention are plant cells. The invention also extends to harvestable parts of a plant, such as but not limited to, seeds, leaves, fruits, flowers, stem cultures, rhizomes, tubers and bulbs.

The term "plant" as used herein encompasses whole plants, ancestors and progeny of the plants and plant parts, including seeds, shoots, stems, roots (including tubers), and plant cells, tissues and organs. The term "plant" also therefore encompasses suspension cultures, embryos, meristematic regions, callus tissue, leaves, gametophytes, sporophytes, pollen, and microspores. Plants that are particularly useful in the methods of the invention include algae, ferns, and all plants which belong to the superfamily *Viridiplantae,* in particular monocotyledonous and dicotyledonous plants, including a fodder or forage legumes, ornamental plants, food crops, trees, or shrubs selected from the list comprising *Abelmoschus* spp., *Acer* spp., *Actinidia* spp., *Agropyron* spp., *Allium* spp., *Amaranthus* spp., *Ananas comosus, Annona* spp., *Apium graveolens, Arabidopsis thaliana, Arachis* spp, *Artocarpus* spp., *Asparagus officinalis, Avena sativa, Averrhoa carambola, Benincasa hispida, Bertholletia excelsea, Beta vulgaris, Brassica* spp., *Cadaba farinosa, Camellia sinensis, Canna indica, Capsicum* spp., *Carica papaya, Carissa macrocarpa, Carthamus tinctorius, Carya* spp., *Castanea* spp., *Cichorium endivia, Cinnamomum spp., Citrullus lanatus, Citrus* spp., *Cocos* spp., Coffea spp., *Cold* spp., *Colocasia esculenta, Corylus spp., Crataegus spp., Cucumis* spp., *Cucurbita* spp., *Cynara* spp., *Daucus carota, Desmodium* spp., *Dimocarpus longan, Dioscorea* spp., *Diospyros* spp., *Echinochloa* spp., *Eleusine coracana, Eriobotrya japonica, Eugenia uniflora, Fagopyrum* spp., *Fagus* spp., *Ficus carica, Fortunella spp., Fragaria* spp., *Ginkgo biloba, Glycine* spp., *Gossypium hirsutum, Helianthus* spp., *Hibiscus* spp., *Hordeum* spp., *Ipomoea batatas, Juglans* spp., *Lactuca sativa, Lathyrus* spp., *Lemna spp., Lens culinaris, Linum usitatissimum, Litchi chinensis, Lotus* spp., *Luffa acutangula, Lupinus* spp., *Macrotyloma* spp., *Malpighia emarginata, Malus* spp., *Mammea americana, Mangifera indica, Manihot* spp., *Manilkara zapota, Medicago sativa, Melilotus* spp., *Mentha* spp., *Momordica* spp., *Morus nigra, Musa* spp., *Nicotiana* spp., *Olea* spp., *Opuntia* spp., *Omithopus* spp., *Oryza* spp., *Panicum miliaceum, Passiflora edulis, Pastinaca sativa, Persea* spp., *Petroselinum crispum, Phaseolus* spp., *Phoenix* spp., *Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Rubus* spp., *Saccharum* spp., *Sambucus* spp., *Secale cereale, Sesamum* spp., *Solanum* spp., *Sorghum bicolor, Spinacia* spp., *Syzygium* spp., *Tamarindus indica, Theobroma cacao, Trifolium* spp., *Triticosecale rimpaui, Triticum* spp., *Vaccinium* spp., *Vicia* spp., *Vigna spp., Vitis* spp., *Zea mays, Zizania palustris, Ziziphus* spp., amongst others.

According to a preferred feature of the present invention, the plant is a crop plant, such as soybean, sunflower, cahola, alfalfa, rapeseed or cotton. Further preferably, the plant according to the present invention is a monocotyledonous plant, such as sugarcane. Most preferably, the plant is a cereal, such as rice, maize, wheat, sorghum, millet or barley.

Advantageously, performance of the methods according to the present invention results in plants having improved growth characteristics, selected from one or more of: an increase in area, an increase in the number of panicles, an increase in height, an increase in the number of seeds, an increase in the number of filled seeds, an increase in the total weight of seeds, an increase in thousand kernel weight (TKW) and an increase in harvest index, each relative to corresponding wild type plants.

The term "increased yield" as defined herein encompasses an increase in biomass (weight) in one or more parts of a plant, particularly aboveground (harvestable) parts, increased root biomass or increased biomass of any other harvestable part, relative to the biomass of the corresponding parts of corresponding wild-type plants. The term also encompasses an increase in seed yield, which includes an increase in the biomass of the seed (seed weight) and which may be an increase in the seed weight per plant or on an individual seed basis, and/or an increase in the number of (filled) seeds and/or in the size of the seeds and/or an increase in seed volume, each relative to corresponding wild-type plants. An increase in seed size and/or volume may also influence the composition of seeds. An increase in seed yield could be due to an increase in the number and/or size of flowers. An increase in yield might also increase the harvest index, which is expressed as a ratio of the total biomass over the yield of harvestable parts, such as seeds. An increase in yield may also increase the thousand kernel weight (TKW) which is extrapolated from the total weight of the number of filled seeds. An increase in TKW may result from an increased seed size and/or seed weight.

Taking corn as an example, a yield increase may be manifested as one or more of the following: increase in the number of plants per hectare or acre, an increase in the number of ears per plant, an increase in the number of rows, number of kernels per row, kernel weight, thousand kernel weight, ear length/diameter, among others. Taking rice as an example, a yield increase may be manifested by an increase in one or more of the following: number of plants per hectare or acre, number of panicles per plant, number of spikelets per panicle, number of flowers per panicle, increase in the seed filling rate, increase in thousand kernel weight, among others. An increase in yield may also result in modified architecture, or may occur as a result of modified architecture.

Since the transgenic plants according to the present invention have increased yield, it is apparent that these plants exhibit an increased growth rate (during at least part of their life cycle), relative to the growth rate of corresponding wild type plants. The increased growth rate may be specific to one or more parts of a plant (including seeds), or throughout the whole plant. A plant having increased growth rate may even exhibit early flowering. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest time of a plant allowing plants to be harvested sooner than would otherwise be possibie. If the growth rate is sufficiently increased, it may allow for the sowing of further seeds of the same plant species (for example sowing and harvesting of rice plants followed by sowing and harvesting of further rice plants all within one conventional growing period) or of different plants species (for example the sowing and harvesting of rice plants followed by, for example, the sowing and optional harvesting of soy bean, potatoes or any other suitable plant), thereby increasing the annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The faster rate of growth may be determined by deriving various parameters from growth curves derived from growth experiments, such parameters may be: T-Mid (the time taken for plants to reach 50% of their maximal size) and T-90 (time taken for plants to reach 90% of their maximal size).

According to a preferred feature of the present invention, performance of the methods of the invention result in plants having modified growth rate. Therefore, according to the present invention, there is provided a method for increasing the growth rate of plants, which method comprises increasing expression in a plant of a B-type CDK nucleic acid/gene encoding a B-type CDK protein comprising: (i) a PPTALRE motif with no mismatches or with a mismatch at position 2 and/or 4 from left to right; (ii) a catalytic kinase domain; and (iii) T-loop activation kinase domain. An increase in growth rate is demonstrated in the examples by the reduced time taken for transgenic plants to reach maturity than control plants.

An increase in yield also encompasses a better performance of the plant under non-stress conditions as well as under stress conditions compared to wild-type plants. Plants typically respond to exposure to stress by growing more slowly. However, since the transgenic plants according to the present invention have increased yield and increased growth rate, it is apparent that transgenic plants will also grow faster during stress conditions than corresponding wild type plants also exposed to the same stress conditions. The stress conditions will typically be the everyday biotic and/or abiotic (environmental) stresses to which a plant may be exposed. Typical abiotic or environmental stresses include temperature stresses caused by atypical hot or cold/freezing temperatures; salt stress; water stress (drought or excess water). Abiotic stresses may also be caused by chemicals. Biotic stresses as typically those stresses caused by pathogens, such as bacteria, viruses, fungi and insects.

"Modified architecture" as defined herein includes any a change in the appearance of any one or more of the leaves, shoots, stems, tillers, inflorescence (for monocotyledonous and dicotyledonous plants), panicles, pollen, ovule, seed, embryo, endosperm, seed coat and aleurone.

According to a preferred feature of the present invention, performance of the methods according to the present invention result in plants having modified architecture. The modified architecture is manifested by at least one of: an increase in aboveground area, an increase in the number of panicles and an increase in height. Therefore, according to the present invention, there is provided a method for modifying the architecture of plants, comprising increasing expression in a plant of a B-type CDK nucleic acid/gene encoding a B-type CDK protein comprising: (i) a PPTALRE motif with no mismatches or with a mismatch at position 2 and/or 4 from left to rights (ii) a catalytic kinase domain: and (iii) T-loop activation kinase domain .

The methods according to the present invention result in plants having improved growth characteristics, as described hereinbefore. These advantageous growth characteristics may be combined with other economically advantageous traits, such as further yield-enhancing traits, tolerance to various stresses, traits modifying various architectural features and/or biochemical and/or physiological features.

According to a further embodiment of the present invention, the use of a B-type CDK nucleic acid/gene encoding a B-type CDK protein comprising, (i) a PPTALRE motif with no mismatches or with a mismatch at position 2 and/or 4 from left to right; (ii) a catalytic kinase domain; and (iii) T-loop activation kinase domain, is provided. For example, B-type CDKs may be used in breeding programmes. The nucleic acid sequence may be on a chromosome, or a part thereof, comprising at least the nucleic acid sequence encoding the B-type CDK protein and preferably also one or more related family members. In an example of such a breeding programme, a DNA marker is identified which may be genetically linked to a gene capable of modulating expression of a nucleic acid encoding a B-type CDK protein in a plant, which gene may be a gene encoding the B-type CDK protein itself or any other gene capable of directly or indirectly influencing expression of a B-type CDK gene and/or activity and/or levels of a B-type CDK protein. This DNA marker may then used in breeding programs to select plants having altered growth characteristics.

Allelic variants of B-type CDKs may be used in particular conventional breeding programmes, such as in marker-assisted breeding. Such breeding programmes sometimes require the introduction of allelic variations in the plants by mutagenic treatment of a plant. One suitable mutagenic method is EMS mutagenesis. Identification of allelic variants then takes place by, for example, PCR. This is followed by a selection step for selection of superior allelic variants of the sequence in question and which give rise to altered growth characteristics of a plant. Selection is typically carried out by monitoring growth performance of plants containing different allelic variants of the sequence in question, for example, different allelic variants of SEQ ID NO: 1. Monitoring growth performance can be done in a greenhouse or in the field. Further optional steps include crossing plants, in which the superior allelic variant was identified, with another plant. This could be used, for example, to make a combination of interesting phenotypic features. Allelic variants also encompass Single Nucleotide Polymorphisms (SNPs), as well as Small Insertion/Deletion Polymorphisms (INDELs). The size of INDELs is usually less than 100 bp). SNPs and INDELs form the largest set of sequence variants in naturally occurring polymorphic strains of most organisms.

The present invention also relates to use of a B-type CDK nucleic acid/gene encoding a B-type CDK protein comprising: (i) a PPTALRE motif with no mismatches or with a mismatch at position 2 and/or 4 from left to right; (ii) a catalytic kinase domain: and (iii) T-loop activation kinase domain improving the growth characteristics of plants, selected from one or more of: an increase in area, an increase in the number of panicles an increase in height, an increase in the number of seeds, an increase in the number of filled seeds, an increase in the total weight of seeds, an increase in thousand kernel weight (TKW) and an increase in harvest index.

The B-type CDK nucleic acid may be isolated from a monocotyledonous or dicotyledonous species, preferably from the family *Brassicaceae,* further preferably from *Arabidopsis thaliana.* The nucleic acid is preferably a class 1 B-type CDK, such as a class 1 B-type CDK selected from the examples of class 1 CDKs shown in Fig. 1, namely, CDK B1;1 from *Arabidopsis thaliana,* CDK B1;2 from *Arabidopsis thaliana,* CDKB1;1 from *Lycopersicon esculentum (tomato),* CDK B1;1 from *Antirrhinum majus,* CDK B1;1 from *Medicago sativa* (alfalfa) and CDK B1 from *Dunaliella tertiolecta.* Further preferably the class 1 B-type CDK is a CDK B1;1 from *Arabidopsis thaliana* or a CDK B1;2 from *Arabidopsis thaliana.* Alternatively, the nucleic acid is preferably a class 2 B-type CDK, such as a class 2 B-type CDK selected from the examples shown in Fig 1, namely, a CDK B2;1 from *Arabidopsis thaliana,* a CDK B2;2 from *Arabidopsis thaliana,* a CDK B2;1 from *Antirrhinum majus,* a CDK B2;1 from *Mesembryanthemum crassifolium,* a CDK B2;1 from *Medicago sativa,* a CDK B2;1 from *Lycopersicon esculentum* and a CDK B 1 from *Oryza sativa.* Further preferably the class 2 B-type CDK is a CDK B2;2 from *Arabidopsis thaliana.*

Most preferably the CDK B1;1 nucleic acid is as represented by SEQ ID NO: 1 or by a portion thereof, or by a nucleic acid sequence capable of hybridising therewith, and wherein the CDK B1;1 protein is as represented by SEQ ID NO: 2, or a homologue, derivative or active fragment thereof. Most preferably the CDK B1;2 nucleic acid is as represented by SEQ ID NO: 3 or by a portion thereof, or by a nucleic acid sequence capable of hybridising therewith, and wherein the CDK B1;2 protein is as represented by SEQ ID NO: 4, or a homologue, derivative or active fragment thereof. Most preferably the CDK B2;2 nucleic acid is as represented by SEQ ID NO: 5 or by a portion thereof, or by a nucleic acid sequence capable of hybridising therewith, and wherein the CDK B2;2 protein is as represented by SEQ ID NO: 6, or a homologue, derivative or active fragment thereof. Each of the CDK B1;1, CDKB1;2 and CDK B2;2 nucleic acids/proteins also encompass the variant nucleic acids and amino acids as described hereinbefore.

The present invention also relates to the use of a B-type CDK nucleic acid/gene and/or to the use of a B-type CDK protein as growth regulators. The B-type CDK nucleic acid sequences hereinbefore described and the B-type CDK amino acid sequences hereinbefore described are clearly useful in modifying the growth characteristics of plants. The sequences would therefore find use as growth regulators or growth stimulators. The present invention also provides a composition comprising a B-type CDK protein as hereinbefore described for the use as a growth regulator.

Conversely, the sequences according to the present invention may also be interesting targets for agrochemical compounds, such as herbicides. Accordingly, the present invention encompasses use of the aforementioned B-type CDK nucleic acids as targets for agrochemical compounds, such as herbicides.

### Description of figures

The present invention will now be described with reference to the following figures in which:
**Fig. 1** is a phylogenetic tree showing the relationship of CDKs from various plants.

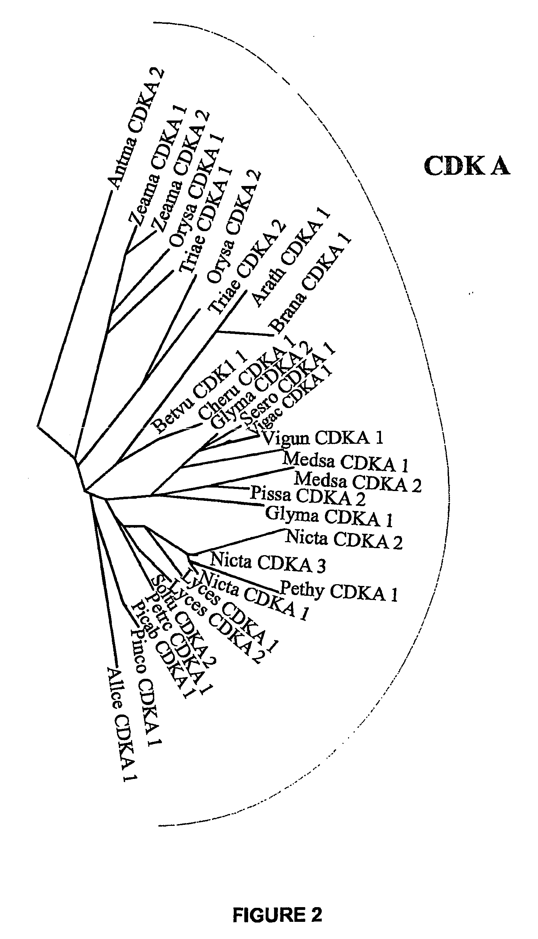

**Fig. 2** is an enlarged view of the CDK A branch of the phylogenetic tree of Fig. 1.
**Fig. 3** is a map of the binary vector for the expression in *Oryza sativa* of an *Arabidopsis thaliana* CDKB1;1 gene under the control of a putative beta-expansin promoter, EXPB8 (SEQ ID NO: 14).
**Fig. 4** is a map of the binary vector for the expression in *Oryza sativa* of an *Arabidopsis thaliana* CDKB1;2 gene under the control of a GOS2 promoter (SEQ ID NO: 15).
**Fig. 5** is a map of the binary vector for the expression in *Oryza sativa* of an *Arabidopsis thaliana* CDKB2;2 gene under the control of a GOS 2 promoter (SEQ ID NO: 15).

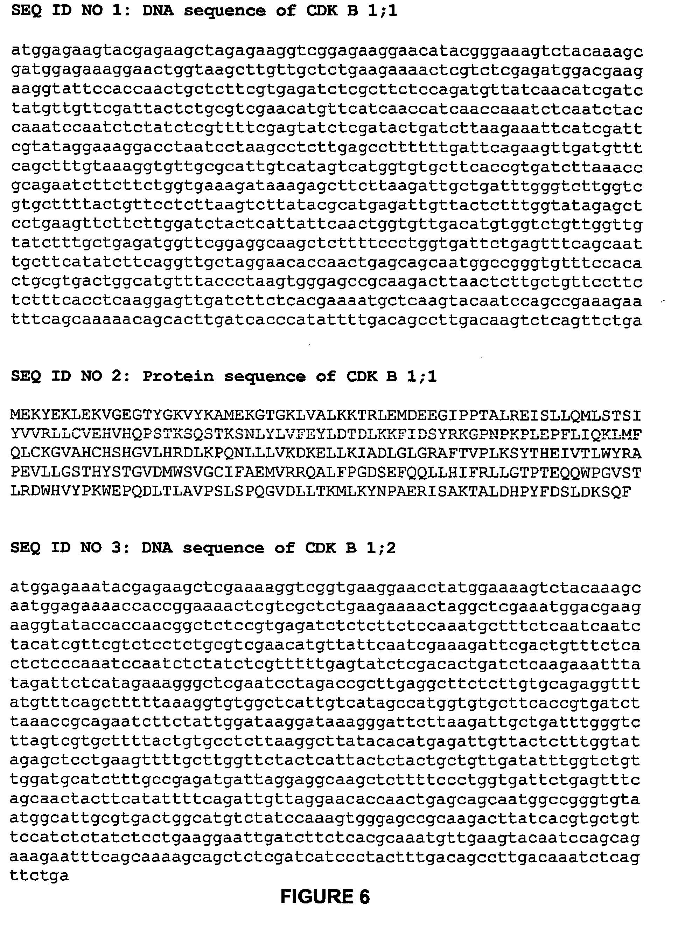

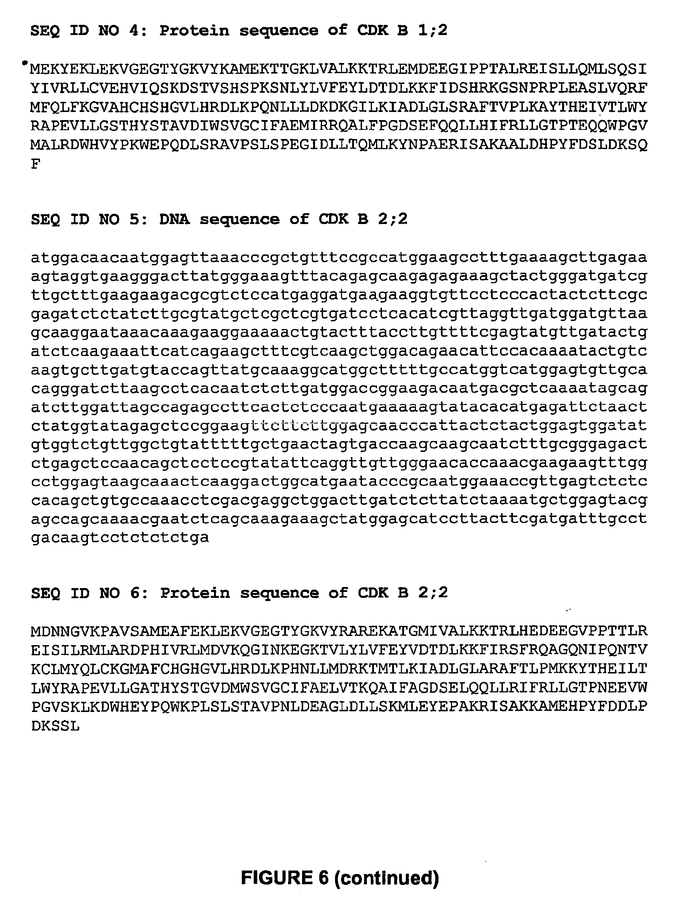

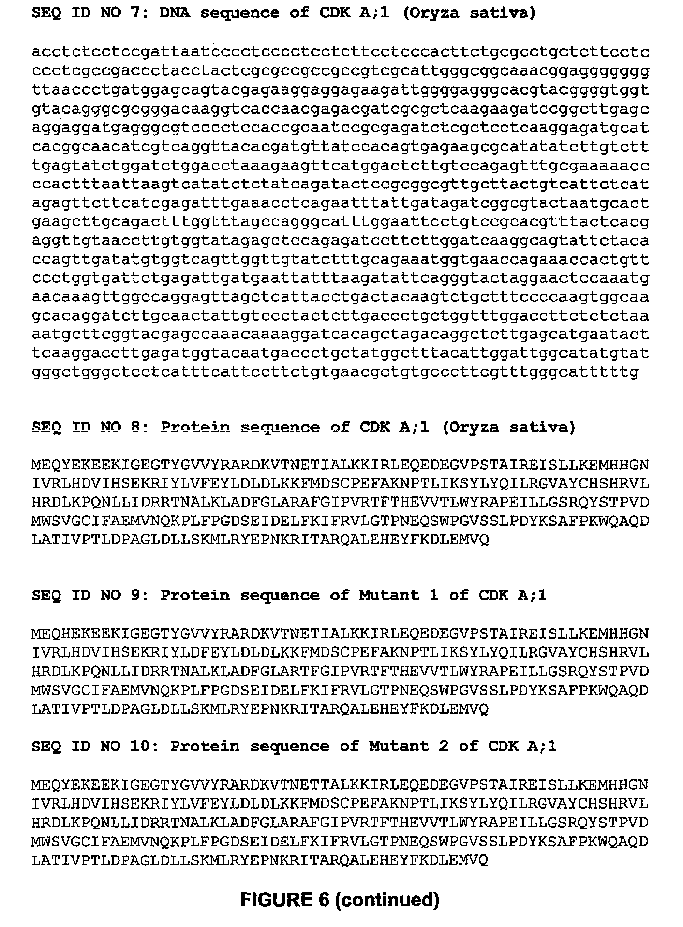

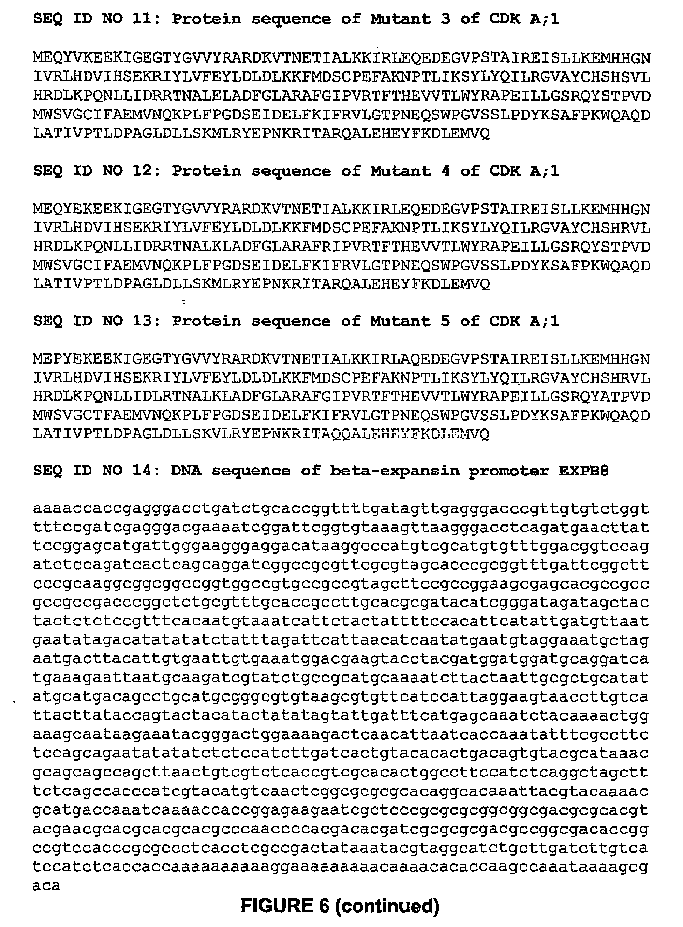

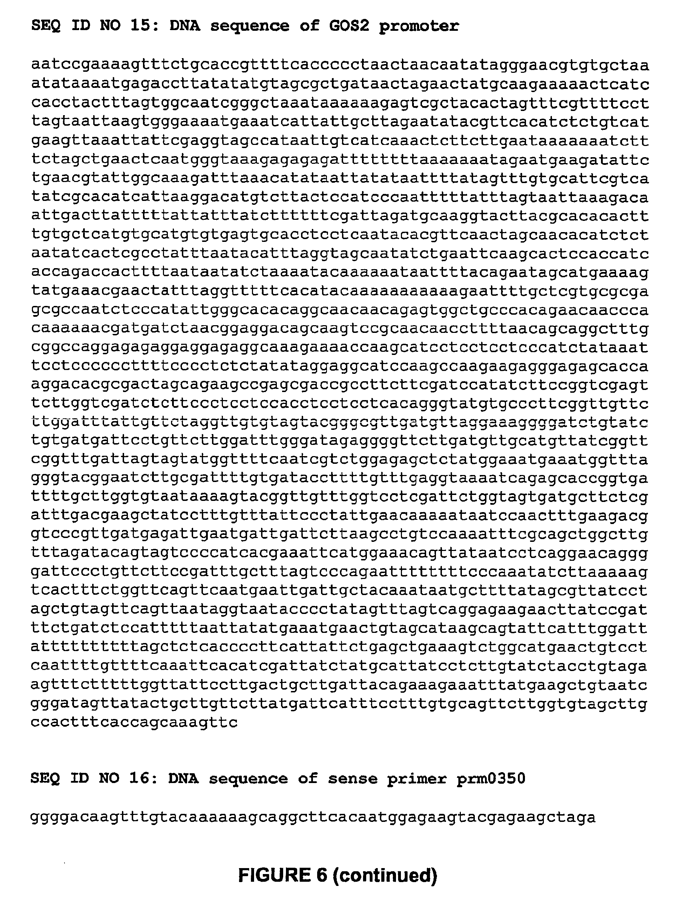

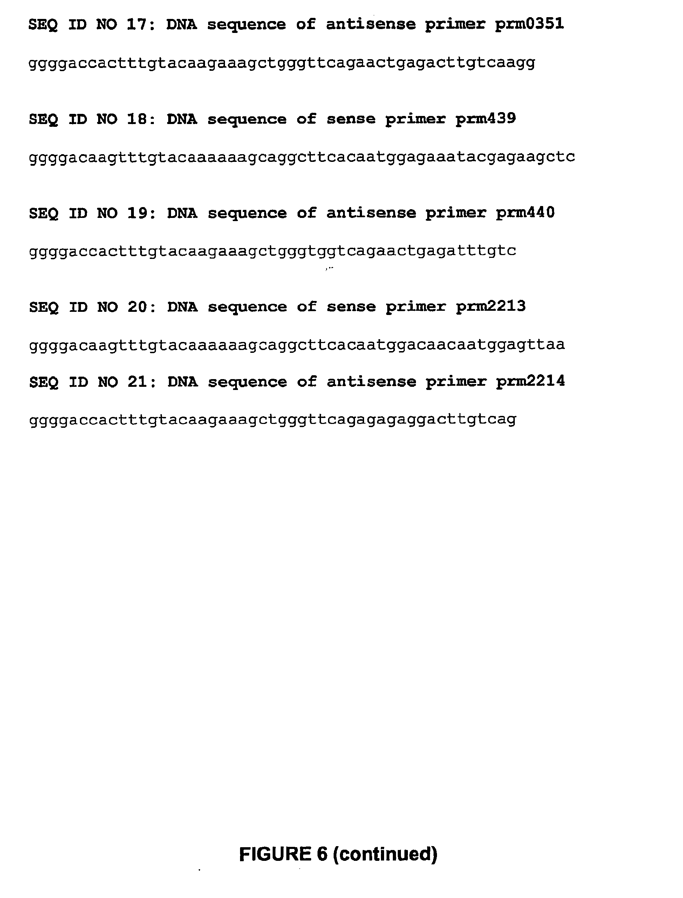

**Fig. 6** details examples of sequences useful in performing the methods according to the present invention.

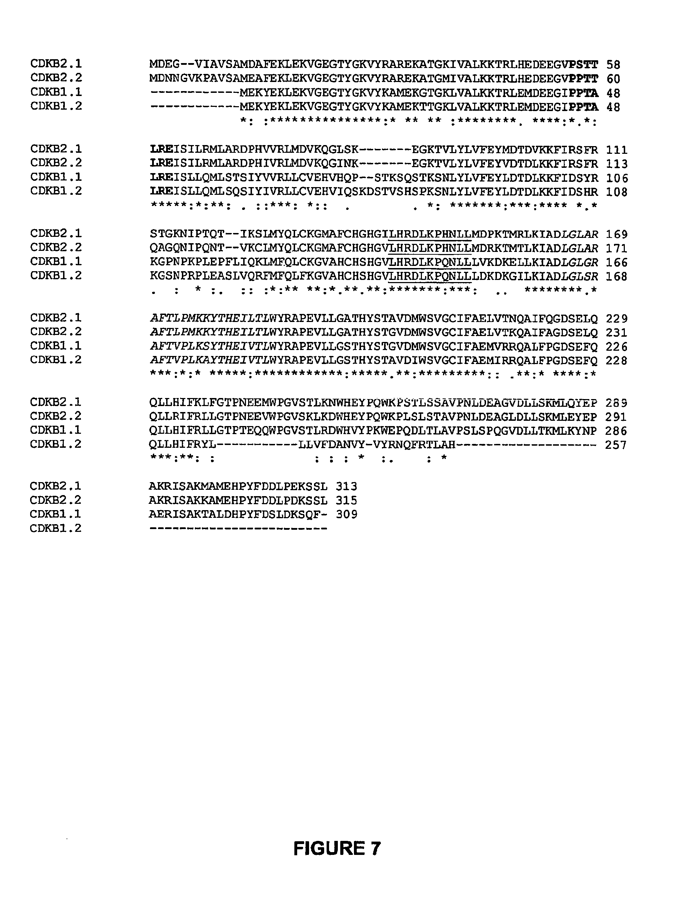

**Fig. 7** is a CLUSTAL W (1.82) multiple sequence alignment for some representative CDK B-type sequences. In bold is a motif found in B-type CDKs; underlined is a catalytic kinase domain and in italics is shown a T-loop activation kinase domain (Magyar et al., 1997 (Plant Cell 9 (2), 223 - 235)).

### Examples

The present invention will now be described with reference to the following examples, which are by way of illustration alone.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, published by BIOS Scientific Publications Ltd (UK) and Blackwell Scientific Publications (UK).

### Example 1: Gene Cloning - CDK B1;1

The *Arabidopsis* CDK B1;1 was amplified by PCR using as a template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb and original number of clones was 1.59x10⁷ cfu. Original titer was determined to be 9.6×10⁵ cfu/ml and, after first amplification, 6x10¹¹ cfu/ml. After plasmid extraction, 200 ng of template was used in a 50 µl PCR mix. Primers prm0350 (sense, start codon in bold, AttB1 site in italic: 5' *GGGGACAAGTTTGTACAAAAAAGCAGGCTTCACA***ATG**GAGAAGTACGAG AAGCTAGA 3') and prm0351 (reverse, complementary, stop codon in bold, AttB2 site in italic: 5' *GGGGACCACTTTGTACAAGAAAGCTGGGT***TCA**GAACTGAGACTTGTCAAGG 3'), which include the AttB sites for Gateway recombination, were used for PCR amplification. PCR was performed using Hifi Taq DNA polymerase in standard conditions. A PCR fragment of 930 bp was amplified and purified also using standard methods. The first step of the Gateway procedure, the BP reaction, was then performed, during which the PCR fragment was recombined *in vivo* with the pDONR201 plasmid to produce, according to Gateway terminology, an "entry clone", p0438. Plasmid pDONR201 was purchased from Invitrogen, as part of the Gateway® technology.

### Example 2: Vector Construction - CDK B1;1

The entry clone p0438 was subsequently used in an LR reaction with p3169, a destination vector used for *Oryza sativa* transformation. This vector contains as functional elements within the T-DNA borders: a plant selectable marker, a plant screenable marker and a Gateway cassette intended for LR *in vivo* recombination with the sequence of interest already cloned in the entry clone. A putative beta-expansin promoter for expression in young expanding tissue is located upstream of this Gateway cassette.

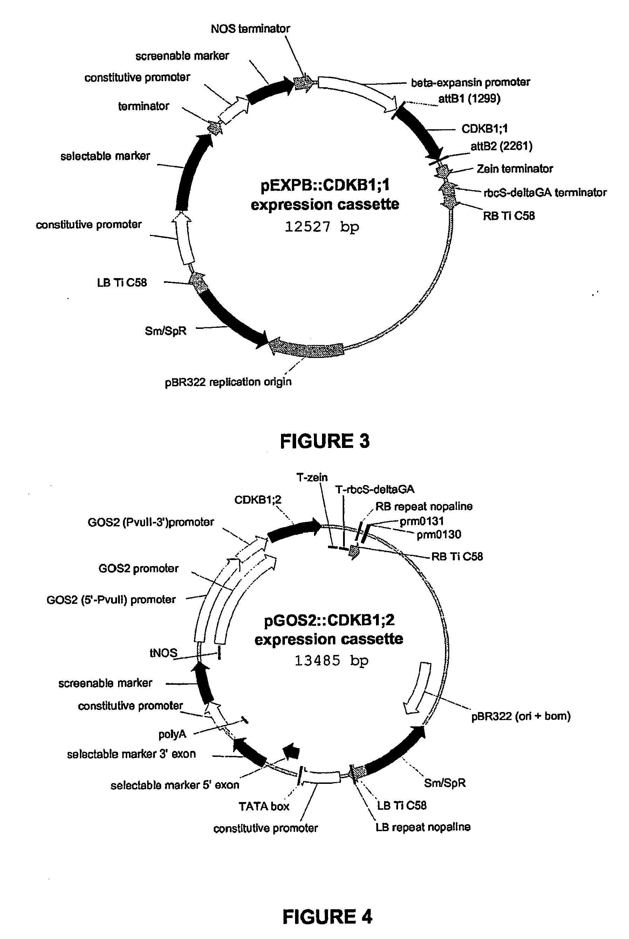

After the LR recombination step, the resulting expression vector as shown in Fig. 3 (CDK B1;1: beta-expansin - overexpression) was transformed into *Agrobacterium* and subsequently to *Oryza sativa* plants. Transformed rice plants were allowed to grow to and then examined for various parameters as described in Example 7.

### Example 3: Gene Cloning - CDK B1;2

The *Arabidopsis* CDK B1;2 was amplified by PCR using as a template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb, and original number of clones was of 1.59×10⁷ cfu. Original titer was determined to be 9.6x10⁵ cfu/ml, after first amplification of 6x10¹¹ cfu/ml. After plasmid extraction, 200 ng of template was used in a 50 µl PCR mix. Primers prm439 (sense, start codon in bold, AttB1 site in italic: 5' *GGGGACAAGTTTGTACAAAAAAGCAGGCTTCACA***ATG**GAGAAATACG AGAAGCTC 3') and prm440 (reverse, complementary, stop codon in bold, AttB2 site in italic: 5' *GGGGACCACTTTGTACAAGAAAGCTGGGTGG***TCA**GAACTGAGATTTGTC 3'), which include the AttB sites for Gateway recombination, were used for PCR amplification. PCR was performed using Hifi Taq DNA polymerase in standard conditions. A PCR fragment of 936bp was amplified and purified also using standard methods. The first step of the Gateway procedure, the BP reaction, was then performed, during which the PCR fragment was recombined *in vivo* with the pDONR201 plasmid to produce, according to the Gateway terminology, an "entry clone", p538. Plasmid pDONR201 was purchased from Invitrogen, as part of the Gateway® technology

### Example 4: Vector Construction CDK B1;2

The entry clone p538 was subsequently used in an LR reaction with p640, a destination vector used for *Oryza sativa* transformation. This vector contains as functional elements within the T-DNA borders: a plant selectable marker; a plant screenable marker; and a Gateway cassette intended for LR *in vivo* recombination with the sequence of interest already cloned in the entry clone. A GOS2 promoter for upregulation was located upstream of this Gateway cassette.

After the LR recombination step, the resulting expression vector as shown in Fig. 4 (CDK B1;2: GOS 2 - overexpression) was transformed into *Agrobacterium* and subsequently to Oryza *sativa* plants. Transformed rice plants were allowed to grow to and then examined for various parameters as described in Example 7.

### Example 5: Gene Cloning - CDK B2;2

The *Arabidopsis* CDKB2;2 was amplified by PCR using as a template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb, and original number of clones was of 1.59x10⁷ cfu. Original titer was determined to be 9.6×10⁵ cfu/ml, after first amplification of 6×10¹¹ cfu/ml. After plasmid extraction, 200 ng of template was used in a 50 µl PCR mix. Primers prm2213 (sense, start codon in bold, AttB1 site in italic: 5' *GGGGACAAGTTTGTACAAAAAAGCAGGCTTCACA***ATG**GACAACAATGG AGTTAA 3') and prm2214 (reverse, complementary, stop codon in bold, AttB2 site in italic: 5' *GGGGACCACTTTGTACAAGAAAGCTGGGT***TCA**GAGAGAGGACTTGTCAG 3'), which include the AttB sites for Gateway recombination, were used for PCR amplification. PCR was performed using Hifi Taq DNA polymerase in standard conditions. A PCR fragment of 948 bp was amplified and purified also using standard methods. The first step of the Gateway procedure, the BP reaction, was then performed, during which the PCR fragment was recombined *in vivo* with the pDONR201 plasmid to produce, according to Gateway terminology, an "entry clone", p2660. Plasmid pDONR201 was purchased from Invitrogen, as part of the Gateway® technology.

### Example 6: Vector Construction - CDK B2;2

The entry clone p2660 was subsequently used in an LR reaction with p640, a destination vector used for *Oryza sativa* transformation. This vector contains as functional elements within the T-DNA borders: a plant selectable marker; a plant screenable marker; and a Gateway cassette intended for LR *in vivo* recombination with the sequence of interest already cloned in the entry clone. A pGOS2 promoter for overexpression was located upstream of this Gateway cassette.

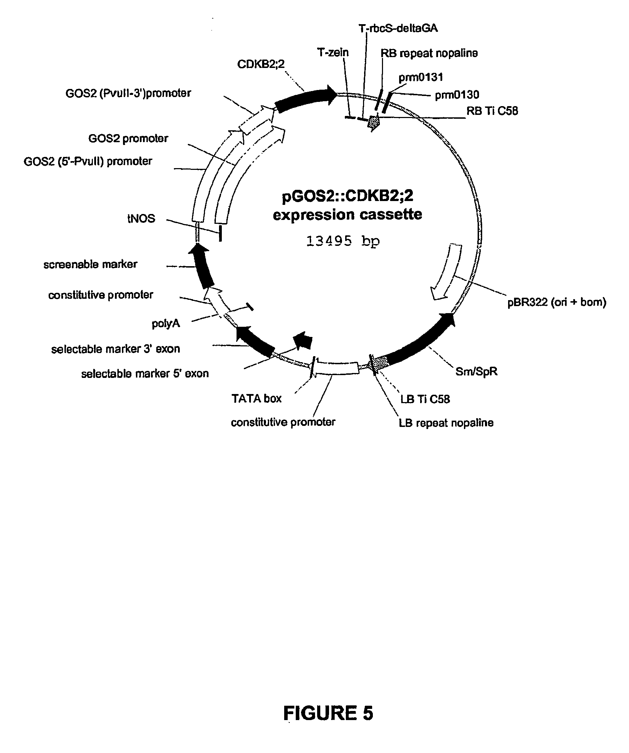

After the LR recombination step, the resulting expression vector as shown in Fig. 5 (CDK B2;2: GOS2 - overexpression) was transformed into *Agrobacterium* and subsequently into *Oryza sativa* plants. Transformed rice plants were allowed to grow to and then examined for various parameters as described in Example 7.

### Example 7: Evaluation and Results

Approximately 15 to 20 independent T0 rice transformants were generated. The primary transformants were transferred from tissue culture chambers to a greenhouse for growing and harvest of T1 seed. 5 events, of which the T1 progeny segregated 3:1 for presence/absence of the transgene, were retained. For each of these events, approximately 10 T1 seedlings containing the transgene (hetero- and homo-zygotes), and approximately 10 T1 seedlings lacking the transgene (nullizygotes), were selected by monitoring visual marker expression.

### Statistical analysis: t-test and F-test

A two factor ANOVA (analysis of variants) was used as statistical model for the overall evaluation of plant phenotypic characteristics. An F-test was carried out on all the parameters measured, for all of the plants of all of the events transformed with the gene of interest. The F-test was carried out to check for an effect of the gene over all the transformation events and to determine the overall effect of the gene or "global gene effect". Significant data, as determined by the value of the F-test, indicates a "gene" effect, meaning that the phenotype observed is caused by more than the presence or position of the gene. In the case of the F-test, the threshold for significance for a global gene effect is set at a 5% probability level.

To check for an effect of the genes within an event, i.e., for a line-specific effect, a t-test was performed within each event using data sets from the transgenic plants and the corresponding null plants. "Null plants" or "Null segregants" are the plants treated in the same way as the transgenic plant, but from which the transgene has segregated. Null plants can also be described as the homozygous negative transformants. The threshold for significance for the t-test is set at 10% probability level. Within one population of transformation events, some events can be under or above this t-test threshold. This is based on the hypothesis that a gene might only have an effect in certain positions in the genome, and that the occurrence of this position-dependent effect is not uncommon. This kind of gene effect may also be referred to as a "line effect of a gene". The p-value is obtained by comparing the t-value to the t-distribution or alternatively, by comparing the F-value to the F-distribution. The p-value stands for the probability that the null hypothesis (null hypothesis being "there is no effect of the transgene") is correct.

### 7.1 Vegetative growth measurements:

The selected T1 plants (approximately 10 with the transgene and approximately 10 without the transgene) were transferred to a greenhouse. Each plant received a unique barcode label to link unambiguously the phenotyping data to the corresponding plant. The selected T1 plants were grown on soil in 10 cm diameter pots under the following environmental settings: photoperiod= 11.5 h, dayiight intensity= 30,000 lux or more, daytime temperature= 28°C or higher, night time temperature= 22°C, relative humidity= 60-70%. Transgenic plants and the corresponding nullizygotes were grown side-by-side at random positions. From the stage of sowing until the stage of maturity each plant was passed several times through a digital imaging cabinet and imaged. At each time point digital images (2048×1536 pixels, 16 million colours) were taken of each plant from at least 6 different angles. The parameters described below were derived in an automated way from all the digital images of all the plants, using image analysis software.

In the tables of results below, each row corresponds to one event. The numeric difference between the positive plants and the negative plants is given (dif) as well as the percentage difference between these plants (% dif). P-value stands for the probability produced by the t-test for each plant line. The last row shows average numbers for all events. In this row, the p-value is the p-value from the F-test.

### (a) Aboveground plant area

Plant aboveground area was determined by counting the total number of pixels from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground.

**Table 2: T1 Aboveground plant area - CDK B1;1**

| **T1 Aboveground plant area - CDK B1;1** | | |
|---|---|---|
| **Line** | **% dif** | **p-value** |
| 1 | -3 | 0.7936 |
| 2 | 11 | 0.4897 |
| 3 | 53 | 0.0011 |
| 4 | 10 | 0.4096 |
| 5 | -2 | 0.8065 |
| **Overall** | **10** | **0.0729** |

As shown in Table 1, line 3 gave a significant increase in the aboveground area for transgenic plants relative to control plants, with a p-value from the t-test of 0.0011. Lines 2 and 4 also showed an increase in aboveground plant area relative to that of control plants. An overall increase of 10% was seen in the aboveground area of transgenic plants compared to control plants.

**Table 3: T1 Aboveground plant area - CDK B1;2**

| **T1 Above ground plant area - CDK B1;2** | | |
|---|---|---|
| **Line** | **% dif** | **p-value** |
| 10 | -5 | 0.6726 |
| 11 | 34 | 0.0002 |
| 12 | 19 | 0.0573 |
| 13 | -6 | 0.4359 |
| **Overall** | **11** | **0.0178** |

As shown in Table 2, lines 11 and 12 gave a significant increase in aboveground plant area with respective p-values from the t-test of 0.0002 and 0.0573. An overall gene effect was also apparent from a p-value of 0.0178 from the F-test. An overall increase of 11% was seen in the aboveground area of transgenic plants compared to control plants. Line 11 was also confirmed in the T2 generation (see Table 4 below) with a 30% increase compared to corresponding nullizygotes and with a p-value from the t test of 0.0002.

**Table 4: T2 Aboveground plant area - CDK B1;2**

| **T2 Aboveground plant area - CDK B1;2** | | |
|---|---|---|
| **Line** | **% dif** | **p-value** |
| 13 | 5 | 0.5379 |
| 12 | -8 | 0.2791 |
| 11 | 30 | 0.0002 |
| **Overall** | **9** | **0.0508** |

### (b) Plant height

Plant height was determined by the distance between the horizontal lines going through the upper pot edge and the uppermost pixel corresponding to a plant part above ground. This value was averaged for the pictures taken on the same time point from the different angles and was converted, by calibration, to a physical distance expressed in mm. Experiments showed that plant height measured this way correlates with plant height measured manually with a ruler.

**Table 5: T1 Height - CDK B1;1**

| **T1 Height - CDK B1;1** | | |
|---|---|---|
| **Line** | **% dif** | **p-value** |
| 1 | -5 | 0.3466 |
| 2 | 1 | 0.8181 |
| 3 | 19 | 0.0045 |
| 4 | 6 | 0.3358 |
| 5 | -2 | 0.7003 |
| **Overall** | **3** | **0.2693** |

The results are shown in Table 5. As shown, line 3 showed a significant increase in plant height relative to corresponding control plants (with a p value from the t-test of 0.0045).

**Table 6: T1 Height - CDK B1;2**

| **T1 Height - CDK B1;2** | | |
|---|---|---|
| **Line** | **% Diff** | **p-value** |
| 13 | -9 | 0.1222 |
| 12 | 10 | 0.1084 |
| 11 | 9 | 0.1022 |
| 10 | -4 | 0.5187 |
| **Overall** | **2** | **0.5361** |

**Table 7: T2 Height - CDK B1;2**

| **T2 Height - CDK B1;2** | | |
|---|---|---|
| **Line** | **% dif** | **p-value** |
| 11 | 9 | 0.019 |
| 12 | -3 | 0.4815 |
| 13 | 5 | 0.2379 |
| **Overall** | **4** | **0.0945** |

Table 6 shows an increase in height in the T1 generation for lines 11 and 12. As shown in Table 7, line 11 showed a significant increase in height in T2 generation plants relative to control plants and gave a p value from the t-test of 0.019.

### 7.2 Seed-related parameter measurements

The mature primary panicles were harvested, bagged, barcode-labelled and then dried for three days in the oven at 37°C. The panicles were then threshed and all the seeds were collected and counted. The filled husks were separated from the empty ones using an airblowing device. The empty husks were discarded and the remaining fraction was counted again. The filled husks were weighed on an analytical balance. This procedure resulted in the set of seed-related parameters described below.

### (c) Total seed number per plant

This was measured by counting the number of husks harvested from a plant.

**Table 8: T1 Total seed number - CDK B1;1**

| **T1 Total Seed Number- CDK B1;1** | | |
|---|---|---|
| **Line** | **% dif** | **p-value** |
| 1 | 10 | 0.4842 |
| 2 | 10 | 0.6685 |
| 3 | 80 | 0.0002 |
| 4 | 3 | 0.8577 |
| 5 | -10 | 0.4176 |
| **Overall** | **12** | **0.081** |

The results are shown in Table 8 above. As shown, line 3 gave a significant increase in the total number of seeds produced by transgenic plants relative to the total number of seeds produced by control plants (with a p value from the t-test of 0.0002).

**Table 9: T2 Total seed number - CDK B1;2**

| **T2 Total Seed Number - CDK B1;2** | | |
|---|---|---|
| **Line** | **% dif** | **p-value** |
| 11 | 32 | 0.0047 |
| 12 | -16 | 0.1356 |
| 13 | -0 | 0.9856 |
| **Overall** | **5** | **0.4425** |

As shown in Table 9, line 11 showed a significant increase (with a p value from the t-test of 0.0047) in the total number of seeds of transgenic plants relative to the total number of seeds of control plants.

### (d) Number of filled seeds

The number of filled seeds was determined by counting the number of filled husks that remained after the separation step.

**Table 10: T1 Number of filled seeds - CDK B1;1**

| **T1 Number of Filled Seeds - CDK B1;1** | | |
|---|---|---|
| **Line** | **%dif** | **p-value** |
| 1 | 20 | 0.3353 |
| 2 | 4 | 0.881 |
| 3 | 67 | 0.0072 |
| 4 | -10 | 0.6329 |
| 5 | -4 | 0.8055 |
| **Overall** | **12** | **0.2026** |

The results are shown in Table 10 above. As shown, line 3 showed a significant increase in the number of filled seeds relative to that of control plants (with a p value of the t test of 0.0072).

**Table 11: T1 Number of filled seeds - CDK B1;2**

| **T1 Number of filled seeds - CDK B1;2** | | |
|---|---|---|
| **Line** | **% dif** | **p-value** |
| 10 | 10 | 0.7578 |
| 11 | 56 | 0.0091 |
| 12 | -18 | 0.3294 |
| 13 | -6 | 0.7196 |
| **Overall** | **14** | **0.3805** |

**Table 12: T2 Number of filled seeds - CDK B1;2**

| **T2 Number of filled seeds - CDK B1;2** | | |
|---|---|---|
| **Line** | **% Diff** | **p-value** |
| 13 | -2 | 0.8861 |
| 12 | -23 | 0.1299 |
| 11 | 45 | 0.0013 |
| **Overall** | **8** | **0.3391** |

As shown in Table 11, line 11 showed an increase in the number of filled seeds relative to control plants with a p value from the t-test of 0.0091. An overall difference of 14% was observed for the number of filled seeds of transgenic plants relative to the number of filled seeds for corresponding control plants. The results of the T2 generation are shown in Table 12, with line 11 performing particularly well.

### (e) Total seed yield per plant

The total seed yield was measured by weighing all filled husks harvested from a plant.

**Table 13: T1 Total weight of seeds - CDK B1;1**

| **T1 Total Weight Seeds - CDK B1;1** | | |
|---|---|---|
| **Line** | **% dif** | **p-value** |
| 1 | 21 | 0.3578 |
| 2 | 28 | 0.4186 |
| 3 | 75 | 0.005 |
| 4 | -9 | 0.6551 |
| 5 | -3 | 0.8182 |
| **Overall** | **16** | **0.1096** |

The results are shown in Table 13 above. As shown, line 3 showed a significant increase (with a p value from the t test of 0.005) in the total weight of seeds of transgenic plants relative to the total weight of the seeds of corresponding non-transgenic plants. An overall increase of 16% was observed for the total weight of the seeds of transgenic plants verses the total weight of the seeds of control plants.

**Table 14: T1: Total weight of seeds - CDK B1;2**

| **T1 Total Weight Seeds - CDK B1;2** | | |
|---|---|---|
| **Line** | **% dif** | **p-value** |
| 10 | 2 | 0.7587 |
| 11 | 46 | 0.0139 |
| 12 | -13 | 0.4276 |
| 13 | -7 | 0.6736 |
| **Overall** | **11** | **0.5841** |

As shown in Table 14, line 11 showed a significant increase in the total weight of the seeds of transgenic plants relative to the total weight of seeds of control plants with a p value from the t-test of 0.0139. The results of the T2 generation are given in Table 15 below, with line 11 performing particularly well.

**Table 15: T2: Total weight of seeds - CDK B1;2**

| **T2 Total Weight Seeds - CDK B1;2** | | |
|---|---|---|
| **Line** | **% Diff** | **p-value** |
| 13 | -6 | 0.7393 |
| 12 | -25 | 0.1509 |
| 11 | 54 | 0.0015 |
| **Overall** | **8** | **0.3694** |

### (f) Harvest index of plants

The harvest index in the present invention is defined as the ratio between the total seed yield and the above ground area (mm²), multiplied by a factor 106.

**Table 16: T 1 Harvest Index - CDK B1;1**

| **T1 Harvest Index - CDK B1;1** | | |
|---|---|---|
| **Line** | **% dif** | **p-value** |
| 1 | 10 | 0.5097 |
| 2 | -2 | 0.8972 |
| 3 | 45 | 0.0201 |
| 4 | -10 | 0.5009 |
| 5 | 0 | 0.9868 |
| **Overall** | **6** | **0.3644** |

The results are shown in Table 16 above. As shown, line 3 showed an increased harvest index for transgenic plants relative to the harvest index of control plants (with a p value from the t-test of 0.0201).

### (g) Thousand Kernel Weight

Thousand Kernel Weight (TKW): this parameter is extrapolated from the number of filled seeds counted and their total weight.

**Table 18: T1: Thousand Kernel Weight (TKW) - CDK B1;2**

| **T2 TKW - CDK B1;2** | | |
|---|---|---|
| **Line** | **% Diff** | **p-value** |
| 13 | -1 | 0.8261 |
| 12 | 5 | 0.1968 |
| 11 | -7 | 0.0648 |
| 10 | -4 | 0.3081 |
| **Overall** | **-2** | **0.3622** |

**Table 19: T2: Thousand Kernel Weight (TKW) - CDK B1;2**

| **T2 TKW - CDK B1;2** | | |
|---|---|---|
| **Line** | **% dif** | **p-value** |
| 11 | 14 | 0.0347 |
| 12 | 1 | 0.8554 |
| 13 | -5 | 0.2525 |
| **Overall** | **3** | **0.4471** |

The results of the T1 generation are shown in Table 18. Table 19 gives the results of the T2 generation. As shown, Line 11 gave a significant increase in the TKW of transgenic plants relative to the TKW of control plants with a p value from the t-test of 0.0347.

### (h) Cycle time - CDK B2;2

Weekly plant area measurements were modelled to obtain a growth curve for each plant. Plant area (in mm²) was plotted against time (in days) and from the resultant growth curve the following parameters were calculated.

**Table 20: T 1 Growth rate - CDK B2;2**

| **T1 Growth Rate (Total Area Cycle Time) - CDK B2;2** | | |
|---|---|---|
| **Line** | **% dif** | **p-value** |
| 20 | -3 | 0.2143 |
| 21 | -4 | 0.0749 |
| 22 | -3 | 0.2296 |
| 23 | 4 | 0.0582 |
| 24 | 1 | 0.4851 |
| 25 | -0 | 0.9693 |
| 26 | -1 | 0.5318 |
| 27 | -4 | 0.0255 |
| **Overall** | **-1** | **0.1062** |

The results are shown in Table 13 above. As shown, lines 21 and 27 gave significant increases in the growth rate of transgenic plants relative to the growth rate of control plants, with increases of 4 days observed in both cases.

### Example 8: Transgenic Corn Expressing a B-type CDK

A B type CDK is cloned under control of a young expanding tissue-preferred or a constitutive promoter in a plant transformation vector suitable for *Agrobacterium*-mediated corn transformation. Vectors and methods for com transformation are selected from those described in any of: EP0604662, EP0672752, EP0971578, EP0955371, EP0558676, Ishida et al. (Nat. Biotechnol. 1996 Jun; 14(6): 745-50); and Frame et al. (Plant Physiol. 2002 May; 129(1): 13-22).

Transgenic plants made by these methods are grown in the greenhouse for T1 seed production. Inheritability and copy number of the transgene is checked by quantitative real-time PCR and Southern blot analysis. Expression levels of the transgene are determined by reverse PCR and Northern analysis. Transgenic lines with single copy insertions of the transgene and with varying levels of transgene expression are selected for T2 seed production.

Progeny seeds are germinated and grown in a greenhouse in conditions adapted for maize (16:8 photoperiod, 26-28°C daytime temperature and 22-24°C night time temperature) as well under water-deficient, nitrogen-deficient, and excess NaCl conditions. In the case of selfing, null segregants from the same parental line, as well as wild type plants of the same cultivar are used as controls. The progeny plants resulting from the selfing or the crosses are evaluated for different biomass and growth parameters, including plant height, stem thickness, number of leaves, total above ground area, leaf greenness, time to maturity, flowering time, ear number, harvesting time. The seeds of these lines are also evaluated for changes in various parameters, such as grain size, total grain yield per plant, and grain quality (starch content, protein content and oil content).

Lines that are most significantly improved compared to corresponding control lines are selected for further field-testing and marker-assisted breeding, with the objective of transferring the field-validated transgenic traits into commercial germplasm. The testing of maize for growth and yield-related parameters in the field is conducted using well-established protocols. Similarly, introgressing specific loci (such as transgene containing loci) from one germplasm into another is also conducted using well-established protocols.

## Claims

1. Method for improving plant growth characteristics selected from one or more of: an increase in area, an increase in the number of panicles, an increase in height, an increase in the number of seeds, an increase in the number of filled seeds, an increase in the total weight of seeds, an increase in thousand kernel weight (TKW) and an increase in harvest index, said method comprising increasing expression in a plant of a nucleic acid encoding a B-type CDK protein by introducing and expressing in a plant a genetic construct comprising a B-type CDK nucleic acid encoding a B-type CDK protein comprising: (i) a PPTALRE motif with no mismatches or with a mismatch at position 2 and/or 4 from left to right; (ii) a catalytic kinase domain; and (iii) a T-loop activation kinase domain.

2. Method according to claim 1, wherein said B-type CDK is derived from a plant, algal or fungal source.

3. Method according to claim 2, wherein said B-type CDK derived from a plant is preferably from a dicotyledonous plant, further preferably from the family *Brassicaceae,* more preferably the nucleic acid sequence is from *Arabidopsis thaliana.*

4. Method according to claim 1 to 3, wherein said B-type CDK is a class 1 B-type CDK, preferably a CDK B1;1 from *Arabidopsis thaliana* or a CDK B1;2 from *Arabidopsis thaliana.*

5. Method according to any one of claims, wherein said B-type CDK is a class 2 B-type CDK, preferably a CDK B2;2 from *Arabidopsis thaliana.*

6. Method according to claim 4, wherein said CDK B1;1 nucleic acid is as represented by SEQ ID NO: 1 or by a portion thereof, or by a nucleic acid sequence hybridising therewith, wherein said portion or hybridising sequence encodes a B-type CDK protein comprising: (i) a PPTALRE motif with no mismatches or with a mismatch at position 2 and/or 4 from left to right; (ii) a catalytic kinase domain; and (iii) a T-loop activation kinase domain.

7. Method according to claim 4, wherein said CDK B1;2 nucleic acid is as represented by SEQ ID NO: 3 or by a portion thereof, or by a nucleic acid sequence hybridising therewith, wherein said portion or hybridising sequence encodes a B-type CDK protein comprising: (i) a PPTALRE motif with no mismatches or with a mismatch at position 2 and/or 4 from left to right; (ii) a catalytic kinase domain; and (iii) a T-loop activation kinase domain.

8. Method according to claim 5, wherein said CDK B2;2 nucleic acid is as represented by SEQ ID NO: 5 or by a portion thereof, or by a nucleic acid sequence hybridising therewith, wherein said portion or hybridising sequence encodes a B-type CDK protein comprising: (i) a PPTALRE motif with no mismatches or with a mismatch at position 2 and/or 4 from left to right; (ii) a catalytic kinase domain; and (iii) a T-loop activation kinase domain.

9. Method according to any of claims 1 to 8, wherein said B-type CDK is selected from:
(a) Alternative splice variants of a B-type CDK nucleic acid/gene;
(b) Allelic variants of a B-type CDK nucleic acid/gene;
(c) Homologues, derivatives and active fragments of a B-type CDK protein;
(d) Mutant B-type CDKs;
wherein said B-type CDK of (a), (b), (c) and (d) each comprise: (i) a PPTALRE motif with no mismatches or with a mismatch at position 2 and/or 4 from left to right; (ii) a catalytic kinase domain; and (iii) a T-loop activation kinase domain.

10. Method according to any one of claims 4,6,9, wherein expression of said CDK B1;1 nucleic acid is driven by a young, expanding tissue-preferred promoter, preferably wherein said promoter is a beta expansin promoter.

11. Method according to any one of claims 4,7,9, wherein expression of said CDK B1;2 nucleic acid and wherein expression of said CDK B2;2 nucleic acid is driven by a constitutive promoter, preferably wherein said promoter is a GOS2 promoter.

12. Plants obtained by a method according to any of claims 1 to 11, wherein said plants comprise a genetic construct comprising:
I. a B-type CDK nucleic acid encoding a B-type CDK protein comprising: (i) a PPTALRE motif with no mismatches or with a mismatch at position 2 and/or 4 from left to right; (ii) a catalytic kinase domain; and (iii) a T-loop activation kinase domain; and
II. one or more control sequences capable of driving expression of the nucleic acid of I, said control sequence comprising a constitutive GOS2 promoter or a beta expansin promoter.

13. Construct comprising:
(a) a B-type CDK gene/nucleic acid encoding a B-type CDK protein comprising: (i) a PPTALRE motif with no mismatches or with a mismatch at position 2 and/or 4 from left to right; (ii) a catalytic kinase domain; and (iii) a T-loop activation kinase domain;
(b) one or more control sequences capable of driving expression of the nucleic acid of (a) said control sequence comprising a constitutive GOS2 promoter a beta expansin promoter; and optionally
(c) a transcription termination sequence.

14. Construct according to claim 13, wherein said nucleic acid of (a) is a CDK B1;2 nucleic acid as represented by SEQ ID NO: 3 or by a portion thereof, or by a nucleic acid sequence capable of hybridising therewith, which nucleic acid encodes a CDK B1;2 protein as represented by SEQ ID NO: 4, or a homologue, derivative or active fragment thereof, wherein said portion or hybridising sequence encodes a B-type CDK protein comprising: (i) a PPTALRE motif with no mismatches or with a mismatch at position 2 and/or 4 from left to right; (ii) a catalytic kinase domain; and (iii) a T-loop activation kinase domain, and wherein said homologue, derivative or active fragment comprises: (i) a PPTALRE motif with no mismatches or with a mismatch at position 2 and/or 4 from left to right; (ii) a catalytic kinase domain; and (iii) a T-loop activation kinase domain.

15. Construct according to claim 13, wherein said nucleic acid of (a) is a CDK B2;2 nucleic acid as represented by SEQ ID NO: 5 or by a portion thereof, or by a nucleic acid sequence capable of hybridising therewith, which nucleic acid encodes a CDK B2;2 protein as represented by SEQ ID NO: 6, or a homologue, derivative or active fragment thereof wherein said portion or hybridising sequence encodes a B-type CDK protein comprising: (i) a PPTALRE motif with no mismatches or with a mismatch at position 2 and/or 4 from left to right; (ii) a catalytic kinase domain; and (iii) a T-loop activation kinase domain, and wherein said homologue, derivative or active fragment comprises: (i) a PPTALRE motif with no mismatches or with a mismatch at position 2 and/or 4 from left to right; (ii) a catalytic kinase domain; and (iii) a T-loop activation kinase domain.

16. Method for the production of a transgenic plant having improved growth characteristics selected from any one or more of: an increase in area, an increase in the number of panicles, an increase in height, an increase in the number of seeds, an increase in the number of filled seeds, an increase in the total weight of seeds, an increase in thousand kernel weight (TKW) and an increase in harvest index, said method comprising the steps of:
(a) introducing into a plant or a plant cell by plant transformation a genetic construct comprising a B-type CDK gene/nucleic acid encoding a B-type CDK protein comprising: (i) a PPTALRE motif with no mismatches or with a mismatch at position 2 and/or 4 from left to right; (ii) a catalytic kinase domain; and (iii) a T-loop activation kinase domain;
(b) cultivating the plant cell under conditions promoting regeneration and mature plant growth.

17. Transgenic plant having improved growth characteristics selected from any one or more of: an increase in area, an increase in the number of panicles, an increase in height, an increase in the number of seeds, an increase in the number of filled seeds, an increase in the total weight of seeds, an increase in thousand kernel weight (TKW) and an increase in harvest index, said plant having increased expression of a B-type CDK nucleic acid encoding a B-type CDK protein relative to corresponding wild type plants and which plant comprises a construct according to any one of claims 13 to 15.

18. Transgenic plant according to claim 17, wherein said plant is a monocotyledonous plant.

## Patentansprüche

1. Verfahren zur Verbesserung von Pflanzenwachstumseigenschaften, ausgewählt aus einer oder mehreren der Folgenden: Flächenvergrößerung, Erhöhung der Rispenzahl, Erhöhung der Länge, Erhöhung der Samenzahl, Erhöhung der Anzahl gefüllter Samen, Erhöhung des Samengesamtgewichts, Erhöhung des Tausendkorngewichts (TKG) und Erhöhung des Harvest Index, wobei das Verfahren umfaßt, dass man die Expression einer Nukleinsäure, die für ein B-Typ-CDK-Protein codiert in einer Pflanze erhöht, und zwar **dadurch**, dass man ein Genkonstrukt umfassend eine B-Typ-CDK-Nukleinsäure, die für ein B-Typ-CDK-Protein codiert, das Folgendes umfaßt: (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne, in eine(r) Pflanze einführt und exprimiert.

2. Verfahren nach Anspruch 1, wobei die B-Typ-CDK aus einem pflanzlichen, Algen- oder pilzlichen Ausgangsmaterial stammt.

3. Verfahren nach Anspruch 2, wobei die aus einer Pflanze abstammende B-Typ-CDK vorzugsweise aus einer dikotylen Pflanze, weiter bevorzugt aus der Familie *Brassicaceae* stammt, stärker bevorzugt stammt die Nukleinsäuresequenz aus *Arabidopsis thaliana.*

4. Verfahren nach Anspruch 1 bis 3, wobei es sich bei der B-Typ-CDK um eine B-Typ-CDK der Klasse 1, vorzugsweise eine CDK-B1;1 aus *Arabidopsis thaliana* oder um eine CDK-B1;2 aus *Arabidopsis thaliana* handelt.

5. Verfahren nach einem der Ansprüche 1 bis 3, wobei es sich bei der B-Typ-CDK um eine B-Typ-CDK der Klasse 2, vorzugsweise um eine CDK-B2;2 aus *Arabidopsis thaliana,* handelt.

6. Verfahren nach Anspruch 4, wobei die CDK-B1;1-Nukleinsäure gemäß SEQ ID Nr.:1 oder gemäß einem Abschnitt davon oder gemäß einer Nukleinsäuresequenz, die hiermit hybridisiert, ist, wobei der Abschnitt oder die hybridisierende Sequenz für ein B-Typ-CDK-Protein codiert, das Folgendes umfaßt: (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne.

7. Verfahren nach Anspruch 4, wobei die CDK-B1;2-Nukleinsäure gemäß SEQ ID Nr.:3 oder gemäß einem Abschnitt davon oder gemäß einer Nukleinsäuresequenz, die hiermit hybridisiert, ist, wobei der Abschnitt oder die hybridisierende Sequenz für ein B-Typ-CDK-Protein codiert, das Folgendes umfaßt: (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne.

8. Verfahren nach Anspruch 5, wobei die CDK-B2;2-Nukleinsäure gemäß SEQ ID Nr.:5 oder gemäß einem Abschnitt davon oder gemäß einer Nukleinsäuresequenz, die hiermit hybridisiert, ist, wobei der Abschnitt oder die hybridisierende Sequenz für ein B-Typ-CDK-Protein codiert, das Folgendes umfaßt: (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne.

9. Verfahren nach einem der Ansprüche 1 bis 8, wobei die B-Typ-CDK aus der folgenden Reihe stammt:
(a) alternative Spleißvarianten einer/eines B-Typ-CDK-Nukleinsäure/Gens;
(b) Allelvarianten einer/eines B-Typ-CDK-Nukleinsäure/Gens;
(c) Homologe, Derivate und aktive Fragmente eines B-Typ-CDK-Proteins;
(d) B-Typ-CDK-Mutanten;
wobei die B-Typ-CDK von (a), (b), (c) und (d) jeweils Folgendes umfaßt: eine B-Typ-CDK-Nukleinsäure, (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne.

10. Verfahren nach einem der Ansprüche 4, 6, 9, wobei die Expression der CDK-B1;1-Nukleinsäure von einem Promoter mit Bevorzugung für junge, expandierende Gewebe vorangetrieben wird, vorzugsweise wobei es sich bei dem Promoter um einen Beta-Expansin-Promoter handelt.

11. Verfahren nach einem der Ansprüche 4, 7, 9, wobei die Expression der CDK-B1;2-Nukleinsäure und wobei die Expression der CDK-B2;2-Nukleinsäure von einem konstitutiven Promoter vorangetrieben wird, vorzugsweise wobei es sich bei dem Promoter um einen GOS2-Promoter handelt.

12. Pflanzen, erhalten nach einem Verfahren nach einem der Ansprüche 1 bis 11, wobei die Pflanzen ein Genkonstrukt umfassen, das Folgendes umfaßt:
I. B-Typ-CDK-Nukleinsäure, die für ein B-Typ-CDK-Protein codiert, das Folgendes umfaßt: (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne; und
II. eine oder mehrere Kontrollsequenzen, die fähig sind, die Expression der Nukleinsäure von I voranzutreiben, wobei die Kontrollsequenz einen konstitutiven GOS2-Promoter oder einen Beta-Expansin-Promoter umfaßt.

13. Genkonstrukt umfassend:
(a) ein/eine B-Typ-CDK-Gen/Nukleinsäure, das/die für ein B-Typ-CDK-Protein codiert, das Folgendes umfaßt: (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne;
(b) eine oder mehrere Kontrollsequenzen, die fähig sind, die Expression der Nukleinsäure von (a) voranzutreiben, wobei die Kontrollsequenz einen konstitutiven GOS2-Promoter oder einen Beta-Expansin-Promoter umfaßt; und gewünschtenfalls
(c) eine Transkriptionsterminationssequenz.

14. Genkonstrukt nach Anspruch 13, wobei es sich bei der Nukleinsäure gemäß (a) um eine CDK-B1;2-Nukleinsäure gemäß SEQ ID Nr.:3 oder gemäß einem Abschnitt davon oder gemäß einer Nukleinsäuresequenz, die fähig ist, hiermit zu hybridisieren, handelt, wobei die Nukleinsäure für ein CDK-B1;2-Protein gemäß SEQ ID Nr.:4 codiert, oder um ein Homolog, Derivat oder aktives Fragment davon handelt, wobei der Abschnitt oder die hybridisierende Sequenz für ein B-Typ-CDK-Protein codiert, das Folgendes umfaßt: eine B-Typ-CDK-Nukleinsäure, (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne, und wobei das Homolog, Derivat oder aktive Fragment Folgendes umfaßt: eine B-Typ-CDK-Nukleinsäure, (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne.

15. Genkonstrukt nach Anspruch 13, wobei es sich bei der Nukleinsäure gemäß (a) um eine CDK-B2;2-Nukleinsäure gemäß SEQ ID Nr.:5 oder gemäß einem Abschnitt davon oder gemäß einer Nukleinsäuresequenz, die fähig ist, hiermit zu hybridisieren, handelt, wobei die Nukleinsäure für ein CDK-B1;2-Protein gemäß SEQ ID Nr.:6 codiert, oder um ein Homolog, Derivat oder aktives Fragment davon handelt, wobei der Abschnitt oder die hybridisierende Sequenz für ein B-Typ-CDK-Protein codiert, das Folgendes umfaßt: eine B-Typ-CDK-Nukleinsäure, (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne, und wobei das Homolog, Derivat oder aktive Fragment Folgendes umfaßt : eine B-Typ-CDK-Nukleinsäure, (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne.

16. Verfahren zur Herstellung einer transgenen Pflanze mit verbesserten Wachstumseigenschaften, ausgewählt von einer oder mehr der Folgenden: Flächenvergrößerung, Erhöhung der Rispenzahl, Erhöhung der Länge, Erhöhung der Samenzahl, Erhöhung der Anzahl gefüllter Samen, Erhöhung des Samengesamtgewichts, Erhöhung des Tausendkorngewichts (TKG) und Erhöhung des Harvest Index, wobei das Verfahren die folgenden Schritte umfaßt:
(a) Einführen eines Genkonstrukts umfassend ein(e) B-Typ-CDK-Gen/Nukleinsäure codierend für ein B-CDK-Protein umfassend: eine B-Typ-CDK-Nukleinsäure, (i) ein PPTALRE-Motif ohne Fehlpaarungen oder mit einer Fehlpaarung in Position 2 und/oder 4 von links nach rechts; (ii) eine katalytische Kinasedomäne, und (iii) eine T-Loop-Aktivierungskinasedomäne, in eine Pflanze oder eine Pflanzenzelle mittels Pflanzentransformation;
(b) Kultivieren der Pflanzenzelle unter Bendingungen, die die Regeneration und das Wachstum der reifen Pflanze fördern.

17. Transgene Pflanze mit verbesserten Wachstumseigenschaften ausgewählt aus einer oder mehreren der Folgenden: Flächenvergrößerung, Erhöhung der Rispenzahl, Erhöhung der Länge, Erhöhung der Samenzahl, Erhöhung der Anzahl gefüllter Samen, Erhöhung des Samengesamtgewichts, Erhöhung des Tausendkorngewichts (TKG) und Erhöhung des Harvest Index, wobei die Pflanze eine im Vergleich zu entsprechenden Wildtyppflanzen erhöhte Expression einer B-Typ-CDK-Nukleinsäure codierend für ein B-Typ-CDK-Protein aufweist und wobei die Pflanze ein Konstrukt nach einem der Ansprüche 13 bis 15 umfaßt.

18. Transgene Pflanze nach Anspruch 17, bei der es sich um eine monokotyle Pflanze handelt.

## Revendications

1. Procédé pour améliorer les caractéristiques de croissance de plantes choisies parmi l'une ou plusieurs de : une augmentation de surface, une augmentation du nombre de panicules, une augmentation de taille, une augmentation du nombre de graines, une augmentation du nombre de graines pleines, une augmentation du poids total de graines, une augmentation du poids de mille grains (TKW) et une augmentation de l'indice de moisson, ledit procédé comprenant l'augmentation de l'expression dans une plante d'un acide nucléique codant pour une protéine CDK de type B en introduisant et en exprimant dans une plante une construction génétique comprenant un acide nucléique CDK de type B codant pour une protéine CDK de type B comprenant : (i) un motif PPTALRE sans mésappariements ou avec un mésappariement à la position 2 et/ou 4 de gauche à droite ; (ii) un domaine kinase catalytique ; et (iii) un domaine kinase d'activation de boucle T.

2. Procédé selon la revendication 1, **caractérisé en ce que** ledit CDK de type B est dérivé d'une source de plante, d'algue ou fongique.

3. Procédé selon la revendication 2, **caractérisé en ce que** ledit CDK de type B dérivé d'une plante est de préférence d'une plante dicotylédone, plus préférablement de la famille *Brassicaceae,* plus préférablement la séquence d'acide nucléique est de *Arabidopsis thaliana.*

4. Procédé selon la revendication 1 à 3 **caractérisé en ce que** ledit CDK de type B est un CDK de type B de classe 1, de préférence un CDK B1;1 de *Arabidopsis* thaliana ou un CDK B1;2 de *Arabidopsis thaliana.*

5. Procédé selon l'une quelconque des revendications 1 à 3 **caractérisé en ce que** ledit CDK de type B est un CDK de type B de classe 2, de préférence un CDK B2;2 de *Arabidopsis thaliana.*

6. Procédé selon la revendication 4, **caractérisé en ce que** ledit acide nucléique CDK B1;1 est comme représenté par SEQ ID NO: 1 ou par une partie de celui-ci, ou par une séquence d'acide nucléique s'hybridant avec celui-ci, **caractérisé en ce que** ladite partie ou séquence d'hybridation code pour une protéine CDK de type B comprenant :
(i) un motif PPTALRE sans mésappariements ou avec un mésappariement à la position 2 et/ou 4 de gauche à droite ; (ii) un domaine kinase catalytique ; et (iii) un domaine kinase d'activation de boucle T.

7. Procédé selon la revendication 4, **caractérisé en ce que** ledit acide nucléique CDK B1;2 est comme représenté par SEQ ID NO: 3 ou par une partie de celui-ci, ou par une séquence d'acide nucléique s'hybridant avec celui-ci, **caractérisé en ce que** ladite partie ou séquence d'hybridation code pour une protéine CDK de type B comprenant :
(i) un motif PPTALRE sans mésappariements ou avec un mésappariement à la position 2 et/ou 4 de gauche à droite ; (ii) un domaine kinase catalytique ; et (iii) un domaine kinase d'activation de boucle T.

8. Procédé selon la revendication 5, **caractérisé en ce que** ledit acide nucléique CDK B2;2 est comme représenté par SEQ ID NO: 5 ou par une partie de celui-ci, ou par une séquence d'acide nucléique s'hybridant avec celui-ci, **caractérisé en ce que** ladite partie ou séquence d'hybridation code pour une protéine CDK de type B comprenant :
(i) un motif PPTALRE sans mésappariements ou avec un mésappariement à la position 2 et/ou 4 de gauche à droite ; (ii) un domaine kinase catalytique ; et (iii) un domaine kinase d'activation de boucle T.

9. Procédé selon l'une quelconque des revendications 1 à 8, **caractérisé en ce que** ledit CDK de type B est choisi parmi :
(a) divers variants d'épissage d'un acide nucléique/gène CDK de type B ;
(b) des variants alléliques d'un acide nucléique/gène CDK de type B ;
(c) des homologues, dérivés et fragments actifs d'une protéine CDK de type B ;
(d) des CDK de type B mutants ;
où lesdits CDK de type B de (a), (b), (c) et (d) comprennent chacun : (i) un motif PPTALRE avec sans mésappariements ou avec un mésappariement à la position 2 et/ou 4 de gauche à droite ; (ii) un domaine kinase catalytique ; et (iii) un domaine kinase d'activation de boucle T.

10. Procédé selon l'une quelconque des revendications 4, 6, 9, **caractérisé en ce que** l'expression dudit acide nucléique CDK B1;1 est contrôlée par un promoteur préféré de tissu jeune en expansion, de préférence où ledit promoteur est un promoteur d'expansine bêta.

11. Procédé selon l'une quelconque des revendications 4, 7, 9, **caractérisé en ce que** l'expression dudit acide nucléique CDK B1;2 et
**caractérisé en ce que** l'expression dudit CDK B2;2 est contrôlée par un promoteur constitutif, de préférence
où ledit promoteur est un promoteur GOS2.

12. Plantes obtenues par un procédé selon l'une quelconque des revendications 1 à 11, **caractérisées en ce que** lesdites plantes comprennent une construction génétique comprenant :
I. un acide nucléique CDK de type B codant pour une protéine CDK de type B comprenant : (i) un motif PPTALRE sans mésappariements ou avec un mésappariement à la position 2 et/ou 4 de gauche à droite ; (ii) un domaine kinase catalytique ; et (iii) un domaine kinase d'activation de boucle T ; et
II. une ou plusieurs séquences capables de contrôler l'expression de l'acide nucléique de I, ladite séquence de contrôle comprenant un promoteur GOS2 constitutif ou un promoteur d'expansine bêta.

13. Construction comprenant :
(a) un gène/acide nucléique CDK de type B codant pour une protéine CDK de type B comprenant : (i) un motif PPTALRE sans mésappariements ou avec un mésappariement à la position 2 et/ou 4 de gauche à droite ; (ii) un domaine kinase catalytique ; et (iii) un domaine kinase d'activation de boucle T ;
(b) une ou plusieurs séquences capables de contrôler l'expression de l'acide nucléique de (a), ladite séquence de contrôle comprenant un promoteur GOS2 constitutif ou un promoteur d'expansine bêta ; et facultativement
(c) une séquence de terminaison de transcription.

14. Construction selon la revendication 13, **caractérisée en ce que** ledit acide nucléique de (a) est un acide nucléique CDK B1;2 comme représenté par SEQ ID NO: 3 ou par une partie de celui-ci, ou par une séquence d'acide nucléique capable de s'hybrider avec celui-ci, ledit acide nucléique code pour une protéine CDK B1;2 comme représentée par SEQ ID NO: 4, ou un homologue, dérivé ou fragment actif de celle-ci, où ladite partie ou séquence d'hybridation code pour une protéine CDK de type B comprenant : (i) un motif PPTALRE sans mésappariements ou avec un mésappariement à la position 2 et/ou 4 de gauche à droite ; (ii) un domaine kinase catalytique ; et (iii) un domaine kinase d'activation de boucle T, et où ledit homologue, dérivé
ou fragment actif comprend : (i) un motif PPTALRE sans mésappariements ou avec un mésappariement à la position 2 et/ou 4 de gauche à droite ; (ii) un domaine kinase catalytique ; et (iii) un domaine kinase d'activation de boucle T.

15. Construction selon la revendication 13, **caractérisée en ce que** ledit acide nucléique de (a) est un acide nucléique CDK B2;2 comme représenté par SEQ ID NO: 5 ou par une partie de celui-ci, ou par une séquence d'acide nucléique capable de s'hybrider avec celui-ci, ledit acide nucléique code pour une protéine CDK B2;2 comme représentée par SEQ ID NO: 6, ou un homologue, dérivé ou fragment actif de celle-ci, où ladite partie ou séquence d'hybridation code pour une protéine CDK de type B comprenant : (i) un motif PPTALRE sans mésappariements ou avec un mésappariement à la position 2 et/ou 4 de gauche à droite ; (ii) un domaine kinase catalytique ; et (iii) un domaine kinase d'activation de boucle T, et où ledit homologue, dérivé
ou fragment actif comprend : (i) un motif PPTALRE sans mésappariements ou avec un mésappariement à la position 2 et/ou 4 de gauche à droite ; (ii) un domaine kinase catalytique ; et (iii) un domaine kinase d'activation de boucle T.

16. Procédé pour la production d'une plante transgénique ayant des caractéristiques de croissance améliorées choisies parmi l'une ou plusieurs de : une augmentation de surface, une augmentation du nombre de panicules, une augmentation de taille, une augmentation du nombre de graines, une augmentation du nombre de graines pleines, une augmentation du poids total de graines, une augmentation du poids de mille grains (TKW) et une augmentation de l'indice de moisson, ledit procédé comprenant les étapes consistant à :
(a) introduire dans une plante ou une cellule de plante par transformation de plante une construction génétique comprenant un gène/acide nucléique CDK de type B codant pour une protéine CDK de type B comprenant : (i) un motif PPTALRE sans mésappariements ou avec un mésappariement à la position 2 et/ou 4 de gauche à droite ; (ii) un domaine kinase catalytique ; et (iii) un domaine kinase d'activation de boucle T ;
(b) cultiver la cellule de plante dans des conditions favorisant la régénération et la croissance de plante mature.

17. Plante transgénique ayant des caractéristiques de croissance améliorées choisies parmi l'une ou plusieurs de : une augmentation de surface, une augmentation du nombre de panicules, une augmentation de taille, une augmentation du nombre de graines, une augmentation du nombre de graines pleines, une augmentation du poids total de graines, une augmentation du poids de mille grains (TKW) et une augmentation de l'indice de moisson, ladite plante ayant une expression augmentée d'un acide nucléique CDK de type B codant pour une protéine CDK de type B par rapport à des plantes de type sauvage correspondantes et ladite plante comprend une construction selon l'une quelconque des revendications 13 à 15.

18. Plante transgénique selon la revendication 17, **caractérisée en ce que** ladite plante une plante monocotylédone.


### SEQUENCE LISTING

<110> CropDesign N.V.
<120> Plants having modified growth characteristics and method for making the same
<130> CD-103-PCT
<150> EP 03077811.2
   <151> 2003-09-05
<160> 21
<170> PatentIn version 3.3
<210> 1
   <211> 930
   <212> DNA
   <213> Arabidopsis thaliana
<400> 1
   atggagaagt acgagaagct agagaaggtc ggagaaggaa catacgggaa agtctacaaa      60
   gcgatggaga aaggaactgg taagcttgtt gctctgaaga aaactcgtct cgagatggac     120
   gaagaaggta ttccaccaac tgctcttcgt gagatctcgc ttctccagat gttatcaaca     180
   tcgatctatg ttgttcgatt actctgcgtc gaacatgttc atcaaccatc aaccaaatct     240
   caatctacca aatccaatct ctatctcgtt ttcgagtatc tcgatactga tcttaagaaa     300
   ttcatcgatt cgtataggaa aggacctaat cctaagcctc ttgagccttt tttgattcag     360
   aagttgatgt ttcagctttg taaaggtgtt gcgcattgtc atagtcatgg tgtgcttcac     420
   cgtgatctta aaccgcagaa tcttcttctg gtgaaagata aagagcttct taagattgct     480
   gatttgggtc ttggtcgtgc ttttactgtt cctcttaagt cttatacgca tgagattgtt     540
   actctttggt atagagctcc tgaagttctt cttggatcta ctcattattc aactggtgtt     600
   gacatgtggt ctgttggttg tatctttgct gagatggttc ggaggcaagc tcttttccct     660
   ggtgattctg agtttcagca attgcttcat atcttcaggt tgctaggaac accaactgag     720
   cagcaatggc cgggtgtttc cacactgcgt gactggcatg tttaccctaa gtgggagccg     780
   caagacttaa ctcttgctgt tccttctctt tcacctcaag gagttgatct tctcacgaaa     840
   atgctcaagt acaatccagc cgaaagaatt tcagcaaaaa cagcacttga tcacccatat     900
   tttgacagcc ttgacaagtc tcagttctga                                      930
<210> 2
   <211> 309
   <212> PRT
   <213> Arabidopsis thaliana
<400> 2
   Met Glu Lys Tyr Glu Lys Leu Glu Lys Val Gly Glu Gly Thr Tyr Gly
   1               5                   10                  15
   Lys Val Tyr Lys Ala Met Glu Lys Gly Thr Gly Lys Leu Val Ala Leu
               20                  25                  30
   Lys Lys Thr Arg Leu Glu Met Asp Glu Glu Gly Ile Pro Pro Thr Ala
           35                  40                  45
   Leu Arg Glu Ile Ser Leu Leu Gln Met Leu Ser Thr Ser Ile Tyr Val
       50                  55                  60
   Val Arg Leu Leu Cys Val Glu His Val His Gln Pro Ser Thr Lys Ser
   65                  70                  75                  80
   Gln Ser Thr Lys Ser Asn Leu Tyr Leu Val Phe Glu Tyr Leu Asp Thr
                   85                  90                  95
   Asp Leu Lys Lys Phe Ile Asp Ser Tyr Arg Lys Gly Pro Asn Pro Lys
               100                 105                 110
   Pro Leu Glu Pro Phe Leu Ile Gln Lys Leu Met Phe Gln Leu Cys Lys
           115                 120                 125
   Gly Val Ala His Cys His Ser His Gly Val Leu His Arg Asp Leu Lys
       130                 135                 140
   Pro Gln Asn Leu Leu Leu Val Lys Asp Lys Glu Leu Leu Lys Ile Ala
   145                 150                 155                 160
   Asp Leu Gly Leu Gly Arg Ala Phe Thr Val Pro Leu Lys Ser Tyr Thr
                   165                 170                 175
   His Glu Ile Val Thr Leu Trp Tyr Arg Ala Pro Glu Val Leu Leu Gly
               180                 185                 190
   Ser Thr His Tyr Ser Thr Gly Val Asp Met Trp Ser Val Gly Cys Ile
           195                 200                 205
   Phe Ala Glu Met Val Arg Arg Gln Ala Leu Phe Pro Gly Asp Ser Glu
       210                 215                 220
   Phe Gln Gln Leu Leu His Ile Phe Arg Leu Leu Gly Thr Pro Thr Glu
   225                 230                 235                 240
   Gln Gln Trp Pro Gly Val Ser Thr Leu Arg Asp Trp His Val Tyr Pro
                   245                 250                 255
   Lys Trp Glu Pro Gln Asp Leu Thr Leu Ala Val Pro Ser Leu Ser Pro
               260                 265                 270
   Gln Gly Val Asp Leu Leu Thr Lys Met Leu Lys Tyr Asn Pro Ala Glu
           275                 280                 285
   Arg Ile Ser Ala Lys Thr Ala Leu Asp His Pro Tyr Phe Asp Ser Leu
       290                 295                 300
   Asp Lys Ser Gln Phe
   305
<210> 3
   <211> 936
   <212> DNA
   <213> Arabidopsis thaliana
<400> 3
   atggagaaat acgagaagct cgaaaaggtc ggtgaaggaa cctatggaaa agtctacaaa      60
   gcaatggaga aaaccaccgg aaaactcgtc gctctgaaga aaactaggct cgaaatggac     120
   gaagaaggta taccaccaac ggctctccgt gagatctctc ttctccaaat gctttctcaa     180
   tcaatctaca tcgttcgtct cctctgcgtc gaacatgtta ttcaatcgaa agattcgact     240
   gtttctcact ctcccaaatc caatctctat ctcgtttttg agtatctcga cactgatctc     300
   aagaaattta tagattctca tagaaagggc tcgaatccta gaccgcttga ggcttctctt     360
   gtgcagaggt ttatgtttca gctttttaaa ggtgtggctc attgtcatag ccatggtgtg     420
   cttcaccgtg atcttaaacc gcagaatctt ctattggata aggataaagg gattcttaag     480
   attgctgatt tgggtcttag tcgtgctttt actgtgcctc ttaaggctta tacacatgag     540
   attgttactc tttggtatag agctcctgaa gttttgcttg gttctactca ttactctact     600
   gctgttgata tttggtctgt tggatgcatc tttgccgaga tgattaggag gcaagctctt     660
   ttccctggtg attctgagtt tcagcaacta cttcatattt tcagattgtt aggaacacca     720
   actgagcagc aatggccggg tgtaatggca ttgcgtgact ggcatgtcta tccaaagtgg     780
   gagccgcaag acttatcacg tgctgttcca tctctatctc ctgaaggaat tgatcttctc     840
   acgcaaatgt tgaagtacaa tccagcagaa agaatttcag caaaagcagc tctcgatcat     900
   ccctactttg acagccttga caaatctcag ttctga                               936
<210> 4
   <211> 311
   <212> PRT
   <213> Arabidopsis thaliana
<400> 4
   Met Glu Lys Tyr Glu Lys Leu Glu Lys Val Gly Glu Gly Thr Tyr Gly
   1               5                   10                  15
   Lys Val Tyr Lys Ala Met Glu Lys Thr Thr Gly Lys Leu Val Ala Leu
               20                  25                  30
   Lys Lys Thr Arg Leu Glu Met Asp Glu Glu Gly Ile Pro Pro Thr Ala
           35                  40                  45
   Leu Arg Glu Ile Ser Leu Leu Gln Met Leu Ser Gln Ser Ile Tyr Ile
       50                  55                  60
   Val Arg Leu Leu Cys Val Glu His Val Ile Gln Ser Lys Asp Ser Thr
   65                  70                  75                  80
   Val Ser His Ser Pro Lys Ser Asn Leu Tyr Leu Val Phe Glu Tyr Leu
                   85                  90                  95
   Asp Thr Asp Leu Lys Lys Phe Ile Asp Ser His Arg Lys Gly Ser Asn
               100                 105                 110
   Pro Arg Pro Leu Glu Ala Ser Leu Val Gln Arg Phe Met Phe Gln Leu
           115                 120                 125
   Phe Lys Gly Val Ala His Cys His Ser His Gly Val Leu His Arg Asp
       130                 135                 140
   Leu Lys Pro Gln Asn Leu Leu Leu Asp Lys Asp Lys Gly Ile Leu Lys
   145                 150                 155                 160
   Ile Ala Asp Leu Gly Leu Ser Arg Ala Phe Thr Val Pro Leu Lys Ala
                   165                 170                 175
   Tyr Thr His Glu Ile Val Thr Leu Trp Tyr Arg Ala Pro Glu Val Leu
               180                 185                 190
   Leu Gly Ser Thr His Tyr Ser Thr Ala Val Asp Ile Trp Ser Val Gly
           195                 200                 205
   Cys Ile Phe Ala Glu Met Ile Arg Arg Gln Ala Leu Phe Pro Gly Asp
       210                 215                 220
   Ser Glu Phe Gln Gln Leu Leu His Ile Phe Arg Leu Leu Gly Thr Pro
   225                 230                 235                 240
   Thr Glu Gln Gln Trp Pro Gly Val Met Ala Leu Arg Asp Trp His Val
                   245                 250                 255
   Tyr Pro Lys Trp Glu Pro Gln Asp Leu Ser Arg Ala Val Pro Ser Leu
               260                 265                 270
   Ser Pro Glu Gly Ile Asp Leu Leu Thr Gln Met Leu Lys Tyr Asn Pro
           275                 280                 285
   Ala Glu Arg Ile Ser Ala Lys Ala Ala Leu Asp His Pro Tyr Phe Asp
       290                 295                 300
   Ser Leu Asp Lys Ser Gln Phe
   305                 310
<210> 5
   <211> 948
   <212> DNA
   <213> Arabidopsis thaliana
<400> 5
   atggacaaca atggagttaa acccgctgtt tccgccatgg aagcctttga aaagcttgag      60
   aaagtaggtg aagggactta tgggaaagtt tacagagcaa gagagaaagc tactgggatg     120
   atcgttgctt tgaagaagac gcgtctccat gaggatgaag aaggtgttcc tcccactact     180
   cttcgcgaga tctctatctt gcgtatgctc gctcgtgatc ctcacatcgt taggttgatg     240
   gatgttaagc aaggaataaa caaagaagga aaaactgtac tttaccttgt tttcgagtat     300
   gttgatactg atctcaagaa attcatcaga agctttcgtc aagctggaca gaacattcca     360
   caaaatactg tcaagtgctt gatgtaccag ttatgcaaag gcatggcttt ttgccatggt     420
   catggagtgt tgcacaggga tcttaagcct cacaatctct tgatggaccg gaagacaatg     480
   acgctcaaaa tagcagatct tggattagcc agagccttca ctctcccaat gaaaaagtat     540
   acacatgaga ttctaactct atggtataga gctccggaag ttcttcttgg agcaacccat     600
   tactctactg gagtggatat gtggtctgtt ggctgtattt ttgctgaact agtgaccaag     660
   caagcaatct ttgcgggaga ctctgagctc caacagctcc tccgtatatt caggttgttg     720
   ggaacaccaa acgaagaagt ttggcctgga gtaagcaaac tcaaggactg gcatgaatac     780
   ccgcaatgga aaccgttgag tctctccaca gctgtgccaa acctcgacga ggctggactt     840
   gatctcttat ctaaaatgct ggagtacgag ccagcaaaac gaatctcagc aaagaaagct     900
   atggagcatc cttacttcga tgatttgcct gacaagtcct ctctctga                 948
<210> 6
   <211> 315
   <212> PRT
   <213> Arabidopsis thaliana
<400> 6
   Met Asp Asn Asn Gly Val Lys Pro Ala Val Ser Ala Met Glu Ala Phe
   1 5 10 15
   Glu Lys Leu Glu Lys Val Gly Glu Gly Thr Tyr Gly Lys Val Tyr Arg
   20 25 30
   Ala Arg Glu Lys Ala Thr Gly Met Ile Val Ala Leu Lys Lys Thr Arg
   35 40 45
   Leu His Glu Asp Glu Glu Gly Val Pro Pro Thr Thr Leu Arg Glu Ile
   50 55 60
   Ser Ile Leu Arg Met Leu Ala Arg Asp Pro His Ile Val Arg Leu Met
   65                  70                  75                  80
   Asp Val Lys Gln Gly Ile Asn Lys Glu Gly Lys Thr Val Leu Tyr Leu
                   85                  90                  95
   Val Phe Glu Tyr Val Asp Thr Asp Leu Lys Lys Phe Ile Arg Ser Phe
               100                 105                 110
   Arg Gln Ala Gly Gln Asn Ile Pro Gln Asn Thr Val Lys Cys Leu Met
           115                 120                 125
   Tyr Gln Leu Cys Lys Gly Met Ala Phe Cys His Gly His Gly Val Leu
       130                 135                 140
   His Arg Asp Leu Lys Pro His Asn Leu Leu Met Asp Arg Lys Thr Met
   145                 150                 155                 160
   Thr Leu Lys Ile Ala Asp Leu Gly Leu Ala Arg Ala Phe Thr Leu Pro
                   165                 170                 175
   Met Lys Lys Tyr Thr His Glu Ile Leu Thr Leu Trp Tyr Arg Ala Pro
               180                 185                 190
   Glu Val Leu Leu Gly Ala Thr His Tyr Ser Thr Gly Val Asp Met Trp
           195                 200                 205
   Ser Val Gly Cys Ile Phe Ala Glu Leu Val Thr Lys Gln Ala Ile Phe
       210                 215                 220
   Ala Gly Asp Ser Glu Leu Gln Gln Leu Leu Arg Ile Phe Arg Leu Leu
   225                 230                 235                 240
   Gly Thr Pro Asn Glu Glu Val Trp Pro Gly Val Ser Lys Leu Lys Asp
                   245                 250                 255
   Trp His Glu Tyr Pro Gln Trp Lys Pro Leu Ser Leu Ser Thr Ala Val
               260                 265                 270
   Pro Asn Leu Asp Glu Ala Gly Leu Asp Leu Leu Ser Lys Met Leu Glu
           275                 280                 285
   Tyr Glu Pro Ala Lys Arg Ile Ser Ala Lys Lys Ala Met Glu His Pro
       290                 295                 300
   Tyr Phe Asp Asp Leu Pro Asp Lys Ser Ser Leu
   305                 310                 315
<210> 7
   <211> 1115
   <212> DNA
   <213> Oryza sativa
<400> 7
   acctctcctc cgattaatcc cctcccctcc tcttcctccc acttctgcgc ctgctcttcc    60
   tcccctcgcc gaccctacct actcgcgccg ccgccgtcgc attgggcggc aaacggaggg   120
   ggggttaacc ctgatggagc agtacgagaa ggaggagaag attggggagg gcacgtacgg   180
   ggtggtgtac agggcgcggg acaaggtcac caacgagacg atcgcgctca agaagatccg   240
   gcttgagcag gaggatgagg gcgtcccctc caccgcaatc cgcgagatct cgctcctcaa   300
   ggagatgcat cacggcaaca tcgtcaggtt acacgatgtt atccacagtg agaagcgcat   360
   atatcttgtc tttgagtatc tggatctgga cctaaagaag ttcatggact cttgtccaga    420
   gtttgcgaaa aaccccactt taattaagtc atatctctat cagatactcc gcggcgttgc    480
   ttactgtcat tctcatagag ttcttcatcg agatttgaaa cctcagaatt tattgataga    540
   tcggcgtact aatgcactga agcttgcaga ctttggttta gccagggcat ttggaattcc    600
   tgtccgcacg tttactcacg aggttgtaac cttgtggtat agagctccag agatccttct    660
   tggatcaagg cagtattcta caccagttga tatgtggtca gttggttgta tctttgcaga    720
   aatggtgaac cagaaaccac tgttccctgg tgattctgag attgatgaat tatttaagat    780
   attcagggta ctaggaactc caaatgaaca aagttggcca ggagttagct cattacctga    840
   ctacaagtct gctttcccca agtggcaagc acaggatctt gcaactattg tccctactct    900
   tgaccctgct ggtttggacc ttctctctaa aatgcttcgg tacgagccaa acaaaaggat    960
   cacagctaga caggctcttg agcatgaata cttcaaggac cttgagatgg tacaatgacc   1020
   ctgctatggc tttacattgg attggcatat gtatgggctg ggctcctcat ttcattcctt   1080
   ctgtgaacgc tgtgcccttc gtttgggcat ttttg                              1115
<210> 8
   <211> 294
   <212> PRT
   <213> Oryza sativa
<400> 8
   Met Glu Gln Tyr Glu Lys Glu Glu Lys Ile Gly Glu Gly Thr Tyr Gly
   1               5                   10                  15
   Val Val Tyr Arg Ala Arg Asp Lys Val Thr Asn Glu Thr Ile Ala Leu
               20                  25                  30
   Lys Lys Ile Arg Leu Glu Gln Glu Asp Glu Gly Val Pro Ser Thr Ala
           35                  40                  45
   Ile Arg Glu Ile Ser Leu Leu Lys Glu Met His His Gly Asn Ile Val
       50                  55                  60
   Arg Leu His Asp Val Ile His Ser Glu Lys Arg Ile Tyr Leu Val Phe
   65                  70                  75                  80
   Glu Tyr Leu Asp Leu Asp Leu Lys Lys Phe Met Asp Ser Cys Pro Glu
                   85                  90                  95
   Phe Ala Lys Asn Pro Thr Leu Ile Lys Ser Tyr Leu Tyr Gln Ile Leu
               100                 105                 110
   Arg Gly Val Ala Tyr Cys His Ser His Arg Val Leu His Arg Asp Leu
           115                 120                 125
   Lys Pro Gln Asn Leu Leu Ile Asp Arg Arg Thr Asn Ala Leu Lys Leu
       130                 135                 140
   Ala Asp Phe Gly Leu Ala Arg Ala Phe Gly Ile Pro Val Arg Thr Phe
   145                 150                 155                 160
   Thr His Glu Val Val Thr Leu Trp Tyr Arg Ala Pro Glu Ile Leu Leu
                   165                 170                 175
   Gly Ser Arg Gln Tyr Ser Thr Pro Val Asp Met Trp Ser Val Gly Cys
               180                 185                 190
   Ile Phe Ala Glu Met Val Asn Gln Lys Pro Leu Phe Pro Gly Asp Ser
           195                 200                 205
   Glu Ile Asp Glu Leu Phe Lys Ile Phe Arg Val Leu Gly Thr Pro Asn
       210                 215                 220
   Glu Gln Ser Trp Pro Gly Val Ser Ser Leu Pro Asp Tyr Lys Ser Ala
   225                 230                 235                 240
   Phe Pro Lys Trp Gln Ala Gln Asp Leu Ala Thr Ile Val Pro Thr Leu
                   245                 250                 255
   Asp Pro Ala Gly Leu Asp Leu Leu Ser Lys Met Leu Arg Tyr Glu Pro
               260                 265                 270
   Asn Lys Arg Ile Thr Ala Arg Gln Ala Leu Glu His Glu Tyr Phe Lys
           275                 280                 285
   Asp Leu Glu Met Val Gln
       290
<210> 9
   <211> 294
   <212> PRT
   <213> Oryza sativa
<400> 9
   Met Glu Gln His Glu Lys Glu Glu Lys Ile Gly Glu Gly Thr Tyr Gly
   1               5                   10                  15
   Val Val Tyr Arg Ala Arg Asp Lys Val Thr Asn Glu Thr Ile Ala Leu
               20                  25                  30
   Lys Lys Ile Arg Leu Glu Gln Glu Asp Glu Gly Val Pro Ser Thr Ala
           35                  40                  45
   Ile Arg Glu Ile Ser Leu Leu Lys Glu Met His His Gly Asn Ile Val
       50                  55                  60
   Arg Leu His Asp Val Ile His Ser Glu Lys Arg Ile Tyr Leu Asp Phe
   65                  70                  75                  80
   Glu Tyr Leu Asp Leu Asp Leu Lys Lys Phe Met Asp Ser Cys Pro Glu
                   85                  90                  95
   Phe Ala Lys Asn Pro Thr Leu Ile Lys Ser Tyr Leu Tyr Gln Ile Leu
               100                 105                 110
   Arg Gly Val Ala Tyr Cys His Ser His Arg Val Leu His Arg Asp Leu
           115                 120                 125
   Lys Pro Gln Asn Leu Leu Ile Asp Arg Arg Thr Asn Ala Leu Lys Leu
       130                 135                 140
   Ala Asp Phe Gly Leu Ala Arg Thr Phe Gly Ile Pro Val Arg Thr Phe
   145                 150                 155                 160
   Thr His Glu Val Val Thr Leu Trp Tyr Arg Ala Pro Glu Ile Leu Leu
                   165                 170                 175
   Gly Ser Arg Gln Tyr Ser Thr Pro Val Asp Met Trp Ser Val Gly Cys
               180                 185                 190
   Ile Phe Ala Glu Met Val Asn Gln Lys Pro Leu Phe Pro Gly Asp Ser
           195                 200                 205
   Glu Ile Asp Glu Leu Phe Lys Ile Phe Arg Val Leu Gly Thr Pro Asn
       210                 215                 220
   Glu Gln Ser Trp Pro Gly Val Ser Ser Leu Pro Asp Tyr Lys Ser Ala
   225                 230                 235                 240
   Phe Pro Lys Trp Gln Ala Gln Asp Leu Ala Thr Ile Val Pro Thr Leu
                   245                 250                 255
   Asp Pro Ala Gly Leu Asp Leu Leu Ser Lys Met Leu Arg Tyr Glu Pro
               260                 265                 270
   Asn Lys Arg Ile Thr Ala Arg Gln Ala Leu Glu His Glu Tyr Phe Lys
           275                 280                 285
   Asp Leu Glu Met Val Gln
       290
<210> 10
   <211> 294
   <212> PRT
   <213> Oryza sativa
<400> 10
   Met Glu Gln Tyr Glu Lys Glu Glu Lys Ile Gly Glu Gly Thr Tyr Gly
   1               5                   10                  15
   Val Val Tyr Arg Ala Arg Asp Lys Val Thr Asn Glu Thr Thr Ala Leu
               20                  25                  30
   Lys Lys Ile Arg Leu Glu Gln Glu Asp Glu Gly Val Pro Ser Thr Ala
           35                  40                  45
   Ile Arg Glu Ile Ser Leu Leu Lys Glu Met His His Gly Asn Ile Val
       50                  55                  60
   Arg Leu His Asp Val Ile His Ser Glu Lys Arg Ile Tyr Leu Val Phe
   65                  70                  75                  80
   Glu Tyr Leu Asp Leu Asp Leu Lys Lys Phe Met Asp Ser Cys Pro Glu
                   85                  90                  95
   Phe Ala Lys Asn Pro Thr Leu Ile Lys Ser Tyr Leu Tyr Gln Ile Leu
               100                 105                 110
   Arg Gly Val Ala Tyr Cys His Ser His Arg Val Leu His Arg Asp Leu
           115                 120                 125
   Lys Pro Gln Asn Leu Leu Ile Asp Arg Arg Thr Asn Ala Leu Lys Leu
       130                 135                 140
   Ala Asp Phe Gly Leu Ala Arg Ala Phe Gly Ile Pro Val Arg Thr Phe
   145                 150                 155                 160
   Thr His Glu Val Val Thr Leu Trp Tyr Arg Ala Pro Glu Ile Leu Leu
                   165                 170                 175
   Gly Ser Arg Gln Tyr Ser Thr Pro Val Asp Met Trp Ser Val Gly Cys
               180                 185                 190
   Ile Phe Ala Glu Met Val Asn Gln Lys Pro Leu Phe Pro Gly Asp Ser
           195                 200                 205
   Glu Ile Asp Glu Leu Phe Lys Ile Phe Arg Val Leu Gly Thr Pro Asn
       210                 215                 220
   Glu Gln Ser Trp Pro Gly Val Ser Ser Leu Pro Asp Tyr Lys Ser Ala
   225                 230                 235                 240
   Phe Pro Lys Trp Gln Ala Gln Asp Leu Ala Thr Ile Val Pro Thr Leu
                   245                 250                 255
   Asp Pro Ala Gly Leu Asp Leu Leu Ser Lys Met Leu Arg Tyr Glu Pro
               260                 265                 270
   Asn Lys Arg Ile Thr Ala Arg Gln Ala Leu Glu His Glu Tyr Phe Lys
           275                 280                 285
   Asp Leu Glu Met Val Gln
       290
<210> 11
   <211> 294
   <212> PRT
   <213> Oryza sativa
<400> 11
   Met Glu Gln Tyr Val Lys Glu Glu Lys Ile Gly Glu Gly Thr Tyr Gly
   1               5                   10                  15
   Val Val Tyr Arg Ala Arg Asp Lys Val Thr Asn Glu Thr Ile Ala Leu
               20                  25                  30
   Lys Lys Ile Arg Leu Glu Gln Glu Asp Glu Gly Val Pro Ser Thr Ala
           35                  40                  45
   Ile Arg Glu Ile Ser Leu Leu Lys Glu Met His His Gly Asn Ile Val
       50                  55                  60
   Arg Leu His Asp Val Ile His Ser Glu Lys Arg Ile Tyr Leu Val Phe
   65                  70                  75                  80
   Glu Tyr Leu Asp Leu Asp Leu Lys Lys Phe Met Asp Ser Cys Pro Glu
                   85                  90                  95
   Phe Ala Lys Asn Pro Thr Leu Ile Lys Ser Tyr Leu Tyr Gln Ile Leu
               100                 105                 110
   Arg Gly Val Ala Tyr Cys His Ser His Ser Val Leu His Arg Asp Leu
           115                 120                 125
   Lys Pro Gln Asn Leu Leu Ile Asp Arg Arg Thr Asn Ala Leu Glu Leu
       130                 135                 140
   Ala Asp Phe Gly Leu Ala Arg Ala Phe Gly Ile Pro Val Arg Thr Phe
   145                 150                 155                 160
   Thr His Glu Val Val Thr Leu Trp Tyr Arg Ala Pro Glu Ile Leu Leu
                   165                 170                 175
   Gly Ser Arg Gln Tyr Ser Thr Pro Val Asp Met Trp Ser Val Gly Cys
               180                 185                 190
   Ile Phe Ala Glu Met Val Asn Gln Lys Pro Leu Phe Pro Gly Asp Ser
           195                 200                 205
   Glu Ile Asp Glu Leu Phe Lys Ile Phe Arg Val Leu Gly Thr Pro Asn
       210                 215                 220
   Glu Gln Ser Trp Pro Gly Val Ser Ser Leu Pro Asp Tyr Lys Ser Ala
   225                 230                 235                 240
   Phe Pro Lys Trp Gln Ala Gln Asp Leu Ala Thr Ile Val Pro Thr Leu
                   245                 250                 255
   Asp Pro Ala Gly Leu Asp Leu Leu Ser Lys Met Leu Arg Tyr Glu Pro
               260                 265                 270
   Asn Lys Arg Ile Thr Ala Arg Gln Ala Leu Glu His Glu Tyr Phe Lys
           275                 280                 285
   Asp Leu Glu Met Val Gln
       290
<210> 12
   <211> 294
   <212> PRT
   <213> Oryza sativa
<400> 12
   Met Glu Gln Tyr Glu Lys Glu Glu Lys Ile Gly Glu Gly Thr Tyr Gly
   1               5                   10                  15
   Val Val Tyr Arg Ala Arg Asp Lys Val Thr Asn Glu Thr Ile Ala Leu
               20                  25                  30
   Lys Lys Ile Arg Leu Glu Gln Glu Asp Glu Gly Val Pro Ser Thr Ala
           35                  40                  45
   Ile Arg Glu Ile Ser Leu Leu Lys Glu Met His His Gly Asn Ile Val
       50                  55                  60
   Arg Leu His Asp Val Ile His Ser Glu Lys Arg Ile Tyr Leu Val Phe
   65                  70                  75                  80
   Glu Tyr Leu Asp Leu Asp Leu Lys Lys Phe Met Asp Ser Cys Pro Glu
                   85                  90                  95
   Phe Ala Lys Asn Pro Thr Leu Ile Lys Ser Tyr Leu Tyr Gln Ile Leu
               100                 105                 110
   Arg Gly Val Ala Tyr Cys His Ser His Arg Val Leu His Arg Asp Leu
           115                 120                 125
   Lys Pro Gln Asn Leu Leu Ile Asp Arg Arg Thr Asn Ala Leu Lys Leu
       130                 135                 140
   Ala Asp Phe Gly Leu Ala Arg Ala Phe Arg Ile Pro Val Arg Thr Phe
   145                 150                 155                 160
   Thr His Glu Val Val Thr Leu Trp Tyr Arg Ala Pro Glu Ile Leu Leu
                   165                 170                 175
   Gly Ser Arg Gln Tyr Ser Thr Pro Val Asp Met Trp Ser Val Gly Cys
               180                 185                 190
   Ile Phe Ala Glu Met Val Asn Gln Lys Pro Leu Phe Pro Gly Asp Ser
           195                 200                 205
   Glu Ile Asp Glu Leu Phe Lys Ile Phe Arg Val Leu Gly Thr Pro Asn
       210                 215                 220
   Glu Gln Ser Trp Pro Gly Val Ser Ser Leu Pro Asp Tyr Lys Ser Ala
   225                 230                 235                 240
   Phe Pro Lys Trp Gln Ala Gln Asp Leu Ala Thr Ile Val Pro Thr Leu
                   245                 250                 255
   Asp Pro Ala Gly Leu Asp Leu Leu Ser Lys Met Leu Arg Tyr Glu Pro
               260                 265                 270
   Asn Lys Arg Ile Thr Ala Arg Gln Ala Leu Glu His Glu Tyr Phe Lys
           275                 280                 285
   Asp Leu Glu Met Val Gln
       290
<210> 13
   <211> 294
   <212> PRT
   <213> Oryza sativa
<400> 13
   Met Glu Pro Tyr Glu Lys Glu Glu Lys Ile Gly Glu Gly Thr Tyr Gly
   1               5                   10                  15
   Val Val Tyr Arg Ala Arg Asp Lys Val Thr Asn Glu Thr Ile Ala Leu
               20                  25                  30
   Lys Lys Ile Arg Leu Ala Gln Glu Asp Glu Gly Val Pro Ser Thr Ala
           35                  40                  45
   Ile Arg Glu Ile Ser Leu Leu Lys Glu Met His His Gly Asn Ile Val
       50                  55                  60
   Arg Leu His Asp Val Ile His Ser Glu Lys Arg Ile Tyr Leu Val Phe
   65                  70                  75                  80
   Glu Tyr Leu Asp Leu Asp Leu Lys Lys Phe Met Asp Ser Cys Pro Glu
                   85                  90                  95
   Phe Ala Lys Asn Pro Thr Leu Ile Lys Ser Tyr Leu Tyr Gln Ile Leu
               100                 105                 110
   Arg Gly Val Ala Tyr Cys His Ser His Arg Val Leu His Arg Asp Leu
           115                 120                 125
   Lys Pro Gln Asn Leu Leu Ile Asp Leu Arg Thr Asn Ala Leu Lys Leu
       130                 135                 140
   Ala Asp Phe Gly Leu Ala Arg Ala Phe Gly Ile Pro Val Arg Thr Phe
   145                 150                 155                 160
   Thr His Glu Val Val Thr Leu Trp Tyr Arg Ala Pro Glu Ile Leu Leu
                   165                 170                 175
   Gly Ser Arg Gln Tyr Ala Thr Pro Val Asp Met Trp Ser Val Gly Cys
               180                 185                 190
   Thr Phe Ala Glu Met Val Asn Gln Lys Pro Leu Phe Pro Gly Asp Ser
           195                 200                 205
   Glu Ile Asp Glu Leu Phe Lys Ile Phe Arg Val Leu Gly Thr Pro Asn
       210                 215                 220
   Glu Gln Ser Trp Pro Gly Val Ser Ser Leu Pro Asp Tyr Lys Ser Ala
   225                 230                 235                 240
   Phe Pro Lys Trp Gln Ala Gln Asp Leu Ala Thr Ile Val Pro Thr Leu
                   245                 250                 255
   Asp Pro Ala Gly Leu Asp Leu Leu Ser Lys Val Leu Arg Tyr Glu Pro
               260                 265                 270
   Asn Lys Arg Ile Thr Ala Gln Gln Ala Leu Glu His Glu Tyr Phe Lys
           275                 280                 285
   Asp Leu Glu Met Val Gln
       290
<210> 14
   <211> 1243
   <212> DNA
   <213> Oryza sativa
<400> 14
   aaaaccaccg agggacctga tctgcaccgg ttttgatagt tgagggaccc gttgtgtctg      60
   gttttccgat cgagggacga aaatcggatt cggtgtaaag ttaagggacc tcagatgaac     120
   ttattccgga gcatgattgg gaagggagga cataaggccc atgtcgcatg tgtttggacg     180
   gtccagatct ccagatcact cagcaggatc ggccgcgttc gcgtagcacc cgcggtttga     240
   ttcggcttcc cgcaaggcgg cggccggtgg ccgtgccgcc gtagcttccg ccggaagcga     300
   gcacgccgcc gccgccgacc cggctctgcg tttgcaccgc cttgcacgcg atacatcggg     360
   atagatagct actactctct ccgtttcaca atgtaaatca ttctactatt ttccacattc     420
   atattgatgt taatgaatat agacatatat atctatttag attcattaac atcaatatga     480
   atgtaggaaa tgctagaatg acttacattg tgaattgtga aatggacgaa gtacctacga     540
   tggatggatg caggatcatg aaagaattaa tgcaagatcg tatctgccgc atgcaaaatc     600
   ttactaattg cgctgcatat atgcatgaca gcctgcatgc gggcgtgtaa gcgtgttcat     660
   ccattaggaa gtaaccttgt cattacttat accagtacta catactatat agtattgatt     720
   tcatgagcaa atctacaaaa ctggaaagca ataagaaata cgggactgga aaagactcaa     780
   cattaatcac caaatatttc gccttctcca gcagaatata tatctctcca tcttgatcac     840
   tgtacacact gacagtgtac gcataaacgc agcagccagc ttaactgtcg tctcaccgtc     900
   gcacactggc cttccatctc aggctagctt tctcagccac ccatcgtaca tgtcaactcg     960
   gcgcgcgcac aggcacaaat tacgtacaaa acgcatgacc aaatcaaaac caccggagaa    1020
   gaatcgctcc cgcgcgcggc ggcgacgcgc acgtacgaac gcacgcacgc acgcccaacc    1080
   ccacgacacg atcgcgcgcg acgccggcga caccggccgt ccacccgcgc cctcacctcg    1140
   ccgactataa atacgtaggc atctgcttga tcttgtcatc catctcacca ccaaaaaaaa    1200
   aaggaaaaaa aaacaaaaca caccaagcca aataaaagcg aca                      1243
<210> 15
   <211> 2191
   <212> DNA
   <213> Oryza sativa
<400> 15
   aatccgaaaa gtttctgcac cgttttcacc ccctaactaa caatataggg aacgtgtgct     60
   aaatataaaa tgagacctta tatatgtagc gctgataact agaactatgc aagaaaaact    120
   catccaccta ctttagtggc aatcgggcta aataaaaaag agtcgctaca ctagtttcgt    180
   tttccttagt aattaagtgg gaaaatgaaa tcattattgc ttagaatata cgttcacatc    240
   tctgtcatga agttaaatta ttcgaggtag ccataattgt catcaaactc ttcttgaata    300
   aaaaaatctt tctagctgaa ctcaatgggt aaagagagag atttttttta aaaaaataga    360
   atgaagatat tctgaacgta ttggcaaaga tttaaacata taattatata attttatagt    420
   ttgtgcattc gtcatatcgc acatcattaa ggacatgtct tactccatcc caatttttat    480
   ttagtaatta aagacaattg acttattttt attatttatc ttttttcgat tagatgcaag    540
   gtacttacgc acacactttg tgctcatgtg catgtgtgag tgcacctcct caatacacgt    600
   tcaactagca acacatctct aatatcactc gcctatttaa tacatttagg tagcaatatc    660
   tgaattcaag cactccacca tcaccagacc acttttaata atatctaaaa tacaaaaaat    720
   aattttacag aatagcatga aaagtatgaa acgaactatt taggtttttc acatacaaaa    780
   aaaaaaagaa ttttgctcgt gcgcgagcgc caatctccca tattgggcac acaggcaaca    840
   acagagtggc tgcccacaga acaacccaca aaaaacgatg atctaacgga ggacagcaag    900
   tccgcaacaa ccttttaaca gcaggctttg cggccaggag agaggaggag aggcaaagaa    960
   aaccaagcat cctcctcctc ccatctataa attcctcccc ccttttcccc tctctatata   1020
   ggaggcatcc aagccaagaa gagggagagc accaaggaca cgcgactagc agaagccgag   1080
   cgaccgcctt cttcgatcca tatcttccgg tcgagttctt ggtcgatctc ttccctcctc   1140
   cacctcctcc tcacagggta tgtgcccttc ggttgttctt ggatttattg ttctaggttg   1200
   tgtagtacgg gcgttgatgt taggaaaggg gatctgtatc tgtgatgatt cctgttcttg   1260
   gatttgggat agaggggttc ttgatgttgc atgttatcgg ttcggtttga ttagtagtat   1320
   ggttttcaat cgtctggaga gctctatgga aatgaaatgg tttagggtac ggaatcttgc   1380
   gattttgtga taccttttgt ttgaggtaaa atcagagcac cggtgatttt gcttggtgta   1440
   ataaaagtac ggttgtttgg tcctcgattc tggtagtgat gcttctcgat ttgacgaagc   1500
   tatcctttgt ttattcccta ttgaacaaaa ataatccaac tttgaagacg gtcccgttga   1560
   tgagattgaa tgattgattc ttaagcctgt ccaaaatttc gcagctggct tgtttagata   1620
   cagtagtccc catcacgaaa ttcatggaaa cagttataat cctcaggaac aggggattcc   1680
   ctgttcttcc gatttgcttt agtcccagaa ttttttttcc caaatatctt aaaaagtcac   1740
   tttctggttc agttcaatga attgattgct acaaataatg cttttatagc gttatcctag   1800
   ctgtagttca gttaataggt aataccccta tagtttagtc aggagaagaa cttatccgat   1860
   ttctgatctc catttttaat tatatgaaat gaactgtagc ataagcagta ttcatttgga   1920
   ttattttttt tttagctctc accccttcat tattctgagc tgaaagtctg gcatgaactg   1980
   tcctcaattt tgttttcaaa ttcacatcga ttatctatgc attatcctct tgtatctacc   2040
   tgtagaagtt tctttttggt tattccttga ctgcttgatt acagaaagaa atttatgaag   2100
   ctgtaatcgg gatagttata ctgcttgttc ttatgattca tttcctttgt gcagttcttg   2160
   gtgtagcttg ccactttcac cagcaaagtt c                                  2191
<210> 16
   <211> 57
   <212> DNA
   <213> Artificial sequence
<220>
   <223> sense primer: prm0350
<400> 16
   ggggacaagt ttgtacaaaa aagcaggctt cacaatggag aagtacgaga agctaga 57
<210> 17
   <211> 51
   <212> DNA
   <213> Artificial sequence
<220>
   <223> antisense primer: prm0351
<400> 17
   ggggaccact ttgtacaaga aagctgggtt cagaactgag acttgtcaag g 51
<210> 18
   <211> 55
   <212> DNA
   <213> Artificial sequence
<220>
   <223> sense primer: prm439
<400> 18
   ggggacaagt ttgtacaaaa aagcaggctt cacaatggag aaatacgaga agctc 55
<210> 19
   <211> 49
   <212> DNA
   <213> Artificial sequence
<220>
   <223> antisense primer: prm440
<400> 19
   ggggaccact ttgtacaaga aagctgggtg gtcagaactg agatttgtc 49
<210> 20
   <211> 54
   <212> DNA
   <213> Artificial sequence
<220>
   <223> sense primer: prm2213
<400> 20
   ggggacaagt ttgtacaaaa aagcaggctt cacaatggac aacaatggag ttaa 54
<210> 21
   <211> 49
   <212> DNA
   <213> Artificial sequence
<220>
   <223> antisense primer: prm2214
<400> 21
   ggggaccact ttgtacaaga aagctgggtt cagagagagg acttgtcag 49